# EUROPEAN PATENT SPECIFICATION

(11) **EP 3 675 853 B1**
(45) Date of publication and mention of the grant of the patent: **07.09.2022**
(21) Application number: 18766482.6
(22) Date of filing: 28.08.2018
(51) Int. Cl.: A61K 31/445, A61P 9/04, A61P 43/00

(54) **METHODS OF ENHANCING AND/OR STABILIZING CARDIAC FUNCTION IN PATIENTS WITH FABRY DISEASE**
VERFAHREN ZUR VERSTÄRKUNG UND/ODER STABILISIERUNG DER HERZFUNKTION IN PATIENTEN MIT MORBUS FABRY
PROCÉDÉS POUR AMÉLIORER ET/OU STABILISER LA FONCTION CARDIAQUE CHEZ DES PATIENTS ATTEINTS DE LA MALADIE DE FABRY

(30) Priority: 28.08.2017 US 201762550984 P
(43) Date of publication of application: 08.07.2020
(73) Proprietor: Amicus Therapeutics, Inc., Philadelphia, PA 19104 (US)
(72) Inventor: CASTELLI, Jeff, Cranbury, New Jersey 08512 (US); BARTH, Jay, Cranbury, New Jersey 08512 (US); SKUBAN, Nina, Cranbury, New Jersey 08512 (US)
(74) Representative: Miller Sturt Kenyon
(86) International application number: PCT/US2018/048257
(87) International publication number: WO 2019/046244

(56) References cited:
- US-A1- 2016 324 839
- DOMINIQUE P. GERMAIN ET AL: "Treatment of Fabry's Disease with the Pharmacologic Chaperone Migalastat", THE NEW ENGLAND JOURNAL OF MEDICINE, - NEJM -, vol. 375, no. 6, 11 August 2016 (2016-08-11), pages 545-555, XP055527557, US ISSN: 0028-4793, DOI: 10.1056/NEJMoa1510198
- MEHTA A. ET AL ON BEHALF OF THE FABRY OUTCOME SURVEY INVESTIGATORS: "Enzyme replacement therapy with agalsidase alfa in patients with Fabry's disease: an analysis of registry data", LANCET, ELSEVIER, AMSTERDAM, NL, vol. 374, no. 9706, 12 December 2009 (2009-12-12), pages 1986-1996, XP027527330, ISSN: 0140-6736, DOI: 10.1016/S0140-6736(09)61493-8 [retrieved on 2009-12-12]
- Germain Dp Schiffmann: "Cardiac Outcomes With Long-term Migalastat Treatment in Patients With Fabry Disease: Results From Phase 3 Trials", 14th Annual WORLDSymposium, San Diego, CA, 5 February 2018 (2018-02-05), pages LB-18, XP055527467, Retrieved from the Internet: URL:https://www.amicusrx.com/app/uploads/W orld2018/World2018-Amicus-Cardiac-Outcomes -migalastat.pdf [retrieved on 2018-11-27]
- Ales Linhart: "The heart in Fabry disease - Fabry Disease", Oxford PharmaGenesis, 1 January 2006 (2006-01-01), Retrieved from the Internet: URL:https://www.ncbi.nlm.nih.gov/books/NBK 11576/ [retrieved on 2022-06-01]

### SEQUENCE LISTING

<110> Amicus Therapeutics, Inc.
<120> METHODS OF ENHANCING AND/OR STABILIZING CARDIAC FUNCTION IN PATIENTS WITH FABRY DISEASE
<130> AT17-007-PCT
<150> US 62/550,984
   <151> 2017-08-28
<160> 3
<170> PatentIn version 3.5
<210> 1
   <211> 12436
   <212> DNA
   <213> Homo sapiens
<400> 1
   cccttctgta ggggcagaga ggttctactt cattactgcg tctcctggga aggccatcag      60
   gactgctggc taaagtggga accaggactc tttgtgagtt aagaatttgt gtatttatat     120
   gtgtgttata cacatttttt aaaaaactgt aacgacatca ggttgagcag tcgtctccgg     180
   gtggtgaatt atgtgtattt ttaaatttta tactatattg ttatttttca aatgttcgaa     240
   attgaatatg tagattgttg ttatcagcag aaaaataaac attattcaaa tactctattc     300
   agtaaagtaa tttattgggc gcctttgtca agcacgcatt tgcctagatg tgactctaca     360
   gataaaattc acttggggcc tccccttaca gacaatcagg cagtggagac tgagtgcctg     420
   aatggataga ccagcactca gaccactatt ttcagtatct gtttttctta actcagggcc     480
   gtggttttca aacgtttttc gccttacggt cacccttagg gtcccccgag accggcccag     540
   acagacagat atacaaaaac acatacacag tcatgagcgt ccaccatttc cccaccaggc     600
   gcagcacagg cggcttcccg gcactgagat gggggggagg agggagagag cgcgaggggg     660
   gaggggaaag cagagaacga aagaggcgga ggcggccccc gaaccccgct ctggtcttca     720
   tcatcaccac ccctgggtcc ccagttccca cccacacacc aacctctaac gataccgggt     780
   aattttcctc cttcttccct caaacggcta tagcgagacg gtagacgacg accagaacta     840
   cttctgctca cgtaagcgag taatcacgtg agcgcctacg tcatgtgaga tctcggtcac     900
   gtgagcaact ctcggcttaa actcgggatc actaaggtgc cgcacttcct tctggtatgg     960
   aaatagggcg ggtcaatatc aagaaaggaa gagggtgatt ggttagcgga acgtcttacg    1020
   tgactgatta ttggtctacc tctggggata accgtcccag ttgccagaga aacaataacg    1080
   tcattattta ataagtcatc ggtgattggt ccgcccctga ggttaatctt aaaagcccag    1140
   gttacccgcg gaaatttatg ctgtccggtc accgtgacaa tgcagctgag gaacccagaa    1200
   ctacatctgg gctgcgcgct tgcgcttcgc ttcctggccc tcgtttcctg ggacatccct    1260
   ggggctagag cactggacaa tggattggca aggacgccta ccatgggctg gctgcactgg 1320
   gagcgcttca tgtgcaacct tgactgccag gaagagccag attcctgcat caggtatcag 1380
   atattgggta ctcccttccc tttgcttttc catgtgtttg ggtgtgtttg gggaactgga 1440
   gagtctcaac gggaacagtt gagcccgagg gagagctccc ccacccgact ctgctgctgc 1500
   ttttttatcc ccagcaaact gtcccgaatc aggactagcc ctaaactttc tctgtgtgac 1560
   ctttcctggg atgggagtcc ggccagcggc ccctgtttct ttctctctct ctctctctct 1620
   cgttctcctt ctctttctct ttctcttctt tcctctctct ttctctctct ccctgcccgg 1680
   ttctcttttt tcactgctcc ttgcagagca gggccacccc ataggcagtg tgcccaaagt 1740
   agccctgccc ggttctattc agacccttct tgtgaacttc tgctcttcct ctgccgggtg 1800
   ctaaccgtta gaacatctag ggtgggtagg aggaatgggg aactaagatt cgtgccattt 1860
   tttctccttt tggggtcgtg gatttctcgg cagtatctcg agggagttag agagaccata 1920
   aggtcgctga gatctctccc acctcgccca tgagcgtggc atcaggctgg aaggttgaca 1980
   tggaggaact ttatacattt acacctttgc gtgagggttg aggctggatt agataggtat 2040
   tgaacatatc tgaccctcac aatccttatc tgtaaattgg gattacaacc ttttaatttc 2100
   agggagctga caaaaaaaat ctgaaaaata gttcttatct cacacaggtg agttttcaag 2160
   gagataacct atttaaagta catagcacag cgcttgacca ttcaactgcg cttacagagc 2220
   aaatgttcaa tgggaaaatg aatgtaaatc tacaaatctg aatgaatatg tgtatttttc 2280
   tggagagagg atatttacct ttcttcaaat tctcaaaggg ctctgtgatt taaaaaaggt 2340
   taggaatcac tgatagatgt tggtaaaagg tggcagtcac agtacatttc tgtgtccata 2400
   agttattcct atgaatatct ttatagataa agtcaggatg ttggtcagac atcacagaag 2460
   aaattggcct tgtaagtttc atgtgaccct gtggtacagt atgtgtggca attttgccca 2520
   tcacggattt ttttttattg gtatttgcat ctgattataa aactaatgca tgatcattgc 2580
   aaaaaatgta gataaagaag agcaaaatga aaataaagat ttccccccac cgttccacca 2640
   cccagaaata atcatggttt aaatgttaat atacaacctt acaattgttt tctatataaa 2700
   tgaaaacata gatttcttta tttcattatt ttccataaaa aatggatcat gtttatgtca 2760
   tgtttggcta atggcaagac cctggcaccc agtctgggct caaattctgc ctcattgtta 2820
   cttagccctg tgacattggg taaattacac tttttttttt tttttttttt tgagacgggg 2880
   tctcgctctg tcgcccaggc tggagtgcag tggcacgatc tcggctcact gcaagtccgc 2940
   ctcctgggtt cacgccattc ttctgcctca gcctcccgag tagctgggac tacaggcgcc 3000
   tgccaccacg cctggctctt tttttttttt tttttttttt tagtacagac ggggtttcac 3060
   catgttagcc agggtggtct caatctcctg acctcgtgat tcgcccgcct cagcctccca 3120
   aagtgctggt gtgagccacc gtgcccagcc ttactttttt ttttgagagg gggtctcact 3180
   ctgtcaccca ggttggagtg cagtggcgcg atctctgctc agtgcaaact ccacctcccg 3240
   ggtttaagca gttctcctgt cgtagtctcc tgagtagctg ggattacagg cacaccacca 3300
   cggccagcta atttttgtat tttcagtaga gacgggtttc accatgttgc ccaagctggt 3360
   ctcgaactcc tggcctcaag tgatctgccc gccttggcct cccagagtgc tgggattaca 3420
   ggtgtgagcc accgcacccg gcctcttttt tcttttttag tctatcatac cttgcaaata 3480
   cagtggttct tcctatgtgt tggttttgat atttatgtaa tcaaacacat cagtttttcc 3540
   tttctgattt ctgactttgg ggtcatgctg agaaagtcct ttcctacctg aagataatac 3600
   agtatatacg tttcttacta gtatttttgt ggatttttaa aatatttaaa tctttagtcc 3660
   atctgaactt gttcttctat cagaaatgcc acatttaata aataataagt cccatggtat 3720
   cagatggctg gaaggacctc tttcgaaact ttgtttaatt ccattaatct gtgtattctt 3780
   attctaatgc taatagttcc acactagctt cctttatctt ttttttcttt tttttttttt 3840
   ttttgagctg gagtttcgct cttgttgccc aggctggagt acaatgtcac gatctcggtt 3900
   caccgcaacc tccgcctccc aggttcaagc aattctcctg cctcatcctc gcgagtagct 3960
   ggaattacag gcatgcgcca ccacgcctag ctattttgta tttttagtag agatggggtt 4020
   tctccatgtt ggtcaggctg gtctcaaact cccagcctca ggtgatctgc ctgcctcggc 4080
   ctcccaaaat gctgttatta caggcgtgag ccaccacgcc cagccttcat cttttaatga 4140
   atgtacatgt atgtaatctt ttaggtgaac tttttgtaat gttgtgccaa gttccttaaa 4200
   aagccctttt ggaagctggg caggtggcca cgcctgtaat cccagcattt tgggagtctg 4260
   aggcaggtgg atcacttgag gccaggagtt caagactagc ctagccaaaa tgcaaaaccc 4320
   tgtctctact aaagatacaa aaattagccg gatgcgatgg cacatgcctg taatctcagc 4380
   tactcgggag gctgaggtag aagaatcgct tgaaccgggg aggcagaggt tgcagtgagc 4440
   aagatggcgc cactgcactc cagcctgggt gacagaggga gactccatct caaaaaaaaa 4500
   aaaaaaaaaa aagataaaaa ggaaacctaa gtactcttgg gctttgttaa ggattttgtt 4560
   aaatatacaa aggattgcag ggaaaattaa cttattttta atattgagta tgcttatcca 4620
   agagcaaaat aatatttctc catttattca aatcatttag gagcatcata gttttaacat 4680
   atgggccttg cacgtatctt aaatttatct ctaggcattt taggttgttc agttgttctt 4740
   gtgaatggga tctttttctc caaataggat tattgttgat atctgttgat tatgttaact 4800
   ttgtagtttc tgactttact gaactgtctt cttagatcta atactctttt caatttcatc 4860
   atatatttct cattcctatt ttgtttgggg tttttagggc gggaatatta acgggataag 4920
   agagacaaaa gaaaatctgg aaaaacaatt cattttacct tacattgctt gtgattacta 4980
   ccacactatt actgggttgg aaaaaattgt gaaatcccaa ggtgcctaat aaatgggagg 5040
   tacctaagtg ttcatttaat gaattgtaat gattattgga atttctcttt cagtgagaag 5100
   ctcttcatgg agatggcaga gctcatggtc tcagaaggct ggaaggatgc aggttatgag 5160
   tacctctgca ttgatgactg ttggatggct ccccaaagag attcagaagg cagacttcag 5220
   gcagaccctc agcgctttcc tcatgggatt cgccagctag ctaattatgt gagtttatag 5280
   ataatgttct tgttcattca gaggactgta agcacttctg tacagaagct tgtttagaaa 5340
   cagccctcat ggccgggcgt ggtggctcac gctgtaatcc caacactttg ggaggccgag 5400
   gcgggtggat cacctgaggt caagagttca agaccagcct ggccaacatg gtgaaacccc 5460
   aactctatta aaagtacaaa aaattagctg ggcatggtgg tgaacgcctg taaccccagc 5520
   tacttgggag gctgaggcag gagaatcgct tgaacccagg aggtggaagt ttcagtgagc 5580
   tgagatcacg ccattgcact ctagcctggg caacaaaaga gaaactccat ctcaaaaaaa 5640
   aaaacaagga aaaaaagaaa cagccctcat gacacttaga aagtagaata gctggctgtt 5700
   atctgaacat tgaattgtaa ggcttatcag gtggactttg cattccatca gcagacaatt 5760
   tttttttttt tttttttttg agatggagtc tcattctgtc tcccaggctg gagggcagtg 5820
   gtgcgatctc ggctcactgc aagctccacc tcctgggttc atgccattct cctgcctcag 5880
   cctcccaagt agctgggacc acaggcaccc gccaccatgc ccagttaatt ttttgtattt 5940
   ttagtagaga cggggtttca ccatgttagc caagatggtc tcgatctcct gacctcgtga 6000
   tccgcccacc tcggcctccc aaagtgctgg gattacaggc atgagccacc gcgcctagcc 6060
   tacaaatgtt ttgtaatagc tcttgaggcc catcttggag ttctcctttt gctaaaacca 6120
   ctgaactctc taggaggaaa aaggaacttg gttcttgaca tatgtgtgca tgtatttcca 6180
   tataaccttt aggaagctat tgcaatggta ctataaacta gaattttaga agatagaagg 6240
   aaaatattct ggagatcatt gaagagaaat ggagtccaac actagttaaa gatgatgaag 6300
   acagattttt ttttttgacg gagtctcgct ctgtcgccca ggctggagtg cagtggcaca 6360
   atctcagctc actgcaaccc tccacctctt gggttcaagt gattctcctg cctcagcctc 6420
   ccaagtagct gggactacag gcgcacacca ccacgcccgg ctaatttttg tatttttagt 6480
   agagacaagg tttcaccata ttcgccaggc tggtctcgaa ctcctgacct tgtaatccgc 6540
   ccaccttggc ctcccaaagt gctgggatta caggcatgag ccaccacgcc cggccgatga 6600
   agacagattt tattcagtac taccacagta gaggaaagag ccaagttcaa ttccaaatac 6660
   aacaaagaca ggtggagatt tatagccaat gagcagattg agggggtcag tggatggaat 6720
   atttaagaag acatcaaggg tagggagctt cttgctaaag cttcatgtac ttaaacaaga 6780
   agggtggggg atgagggaaa ttgatcagat atcaatggtg gcagtattga cttagcagga 6840
   ttcttgctaa gaggtcttgc taggacagac ataggaagcc aaggtggagg tctagtcgaa 6900
   aagaaggctc atcagagaag tctaactaaa gtttggtcaa gaagagtctt tgtcaaggta 6960
   aatctatcat ttccctcaaa aggtaatttt caggatccca tcaggaagat tagcatggct 7020
   gctagctttc tcctcagttc tgggctatag ctcacatgcc tagtttgaac tagctcagca 7080
   gaactggggg atttattctt tgtcttccaa caaactcatc tggatgattt tgggggtttg 7140
   tggggaaaag cccccaatac ctggtgaagt aaccttgtct cttcccccag cctggaatgg 7200
   ttctctcttt ctgctacctc acgattgtgc ttctacaatg gtgactcttt tcctccctct 7260
   catttcaggt tcacagcaaa ggactgaagc tagggattta tgcagatgtt ggaaataaaa 7320
   cctgcgcagg cttccctggg agttttggat actacgacat tgatgcccag acctttgctg 7380
   actggggagt agatctgcta aaatttgatg gttgttactg tgacagtttg gaaaatttgg 7440
   cagatggtaa tgtttcattc cagagattta gccacaaagg aaagaacttt gaggccatgg 7500
   tagctgagcc aaagaaccaa tcttcagaat tttaaatacc ctgtcacaat actggaaata 7560
   attattctcc atgtgccaga gctcccatct cttctctttc agttcattaa ttaattaatt 7620
   aattcatgta aaatccatgc atacctaacc atagctaata ttgtgcactt ataattcaag 7680
   agggctctaa gagttaatta gtaattgtaa ctctctataa catcatttag gggagtccag 7740
   gttgtcaatc ggtcacagag aaagaagcat cttcattcct gcctttcctc aatatacaca 7800
   ccatctctgc actacttcct cagaacaatc ccagcagtct gggaggtact ttacacaatt 7860
   taagcacaga gcaactgcct gtccctgctg ctagtttaaa catgaacctt ccaggtagcc 7920
   tcttcttaaa atatacagcc ccagctgggc atgatggctc atgcctgtaa tcctagcact 7980
   ttgggaggct gaggcgggtg gattacttga ggtcaggagt tcgagaccac cctggccaac 8040
   atggtgaaac cccatctcta gtaaaaatac aaaaattagc tgactttggt ggcacatgcc 8100
   tgtaatccca gctacttggg aagctgagac agaagagtca cttgaacctg ggaaacagag 8160
   gttgcagtga gccaagatcg caccactgca ctccaccctg gatgacagac tgaaccccat 8220
   ctcaaaaaat taaaataaaa taaaataaaa taactatata tatagcccca gctggaaatt 8280
   catttctttc ccttatttta cccattgttt tctcatacag gttataagca catgtccttg 8340
   gccctgaata ggactggcag aagcattgtg tactcctgtg agtggcctct ttatatgtgg 8400
   ccctttcaaa aggtgagata gtgagcccag aatccaatag aactgtactg atagatagaa 8460
   cttgacaaca aaggaaacca aggtctcctt caaagtccaa cgttacttac tatcatccta 8520
   ccatctctcc caggttccaa ccacttctca ccatccccac tgctgtaatt atagcctaag 8580
   ctaccatcac ctggaaagtc atccttgtgt cttccccttt atttcaccat tcatgtcctg 8640
   tctatcaaca gtccttccac cagtatctct aaaatatctc ctgaatcagc ccacttcctt 8700
   ccatcttcac tacatgcacc ctggccttcc aagctactat cggctctcaa ccagactgct 8760
   gggaccacct gatctctctg cttccactct gtctcaaccc ccatctattt tccaagcagc 8820
   actagagtta tcatattaaa atgtaaatat cagttttttt tttaaagaaa aaaaccctga 8880
   gacttaacag agttataaaa aatataaatg tcatcatcag ttccctgctt aaaaccctta 8940
   actcgcttcc aattgcactt ggaatgaaac caaactgcac tgatccagcc cttgcctgcc 9000
   tccccaaagt ccaaggggtc atggctcttt ccctggctac actggttttc tttctgtccc 9060
   tcaacactgc aagcctattg ctgccccagg gcctttacac ttgctttttt tctgcctaga 9120
   acagttcttc cccaaagatt tttaaagggc cgggctcctt aacattgaag tcgcagacca 9180
   aacgccacat atgcagacag ttcttctcta actactttaa aatagccctc tgtccattca 9240
   ttcttcatca cattaacctg tttaattttc ttctcagagc tccacactat ttggaagtat 9300
   ttgttgactt gttaccatgt ctccccacta gagtgtaagt ttcatgaggg cagggacctt 9360
   gtctgacttt gactgtatct ctcgcatatg gttaagtgtt aaatagttat ttatggaatg 9420
   aatccctatt attccctcat tatctctgca aaatagtctt ttttctcaac atcttaaacc 9480
   tgatatccca cctgcctatc tacaaacttt ttttttgcga cagagtctca ctgtcaccca 9540
   ggctagagtg cagtggcgcc atctcggctc actgcaacct ccgcctcccg ggtttaagcg 9600
   attctcttgc ctcagcctcc cagtagctgg gattataggc gtgcgctacc acatctggct 9660
   aatttttgta tttttagtag agatggtttc accatgttgg ccaggcttgt ctcgaactcc 9720
   tgacctcaga tgatccacct gcctcggcct cccaaagtgc tgggattaca ggcatgagcc 9780
   accgtgccca gcctctacaa actttttatt ccattaacaa actatatgct gggatttaag 9840
   ttttcttaat acttgatgga gtcctatgta attttcgagc ttttaatttt actaagacca 9900
   ttttagttct gattatagaa gtaaattaac tttaagggat ttcaagttat atggcctact 9960
   tctgaagcaa acttcttaca gtgaaaattc attataaggg tttagacctc cttatggaga 10020
   cgttcaatct gtaaactcaa gagaaggcta caagtgcctc ctttaaactg ttttcatctc 10080
   acaaggatgt tagtagaaag taaacagaag agtcatatct gttttcacag cccaattata 10140
   cagaaatccg acagtactgc aatcactggc gaaattttgc tgacattgat gattcctgga 10200
   aaagtataaa gagtatcttg gactggacat cttttaacca ggagagaatt gttgatgttg 10260
   ctggaccagg gggttggaat gacccagata tggtaaaaac ttgagccctc cttgttcaag 10320
   accctgcggt aggcttgttt cctattttga cattcaaggt aaatacaggt aaagttcctg 10380
   ggaggaggct ttatgtgaga gtacttagag caggatgctg tggaaagtgg tttctccata 10440
   tgggtcatct aggtaacttt aagaatgttt cctcctctct tgtttgaatt atttcattct 10500
   ttttctcagt tagtgattgg caactttggc ctcagctgga atcagcaagt aactcagatg 10560
   gccctctggg ctatcatggc tgctccttta ttcatgtcta atgacctccg acacatcagc 10620
   cctcaagcca aagctctcct tcaggataag gacgtaattg ccatcaatca ggaccccttg 10680
   ggcaagcaag ggtaccagct tagacaggta aataagagta tatattttaa gatggcttta 10740
   tatacccaat accaactttg tcttgggcct aaatctattt ttttcccttg ctcttgatgt 10800
   tactatcagt aataaagctt cttgctagaa acattacttt atttccaaaa taatgctaca 10860
   ggatcatttt aatttttcct acaagtgctt gatagttctg acattaagaa tgaatgccaa 10920
   actaacaggg ccacttatca ctagttgcta agcaaccaca ctttcttggt ttttcaggga 10980
   gacaactttg aagtgtggga acgacctctc tcaggcttag cctgggctgt agctatgata 11040
   aaccggcagg agattggtgg acctcgctct tataccatcg cagttgcttc cctgggtaaa 11100
   ggagtggcct gtaatcctgc ctgcttcatc acacagctcc tccctgtgaa aaggaagcta 11160
   gggttctatg aatggacttc aaggttaaga agtcacataa atcccacagg cactgttttg 11220
   cttcagctag aaaatacaat gcagatgtca ttaaaagact tactttaaaa tgtttatttt 11280
   attgccaact actacttcct gtccaccttt ttctccattc actttaaaag ctcaaggcta 11340
   ggtggctcat gcctgtaatc ccagcacttt gggaggctga ggcgggcaga tcacctgagg 11400
   tcgggacttt gagacccgcc tggacaacat ggtgaaaccc catttctaat aaaaatataa 11460
   aaattagcca ggtgtggtgg cgcacctgtg gtcccagcta ctctgggggc tgaggcatga 11520
   gaatcgcttg aacccgggag tggaggttgc attgagctga gatcatgcca cctcactcca 11580
   gcctgggcaa caaagattcc atctcaaaaa aaaaaaaaaa gccaggcaca gtggctcatg 11640
   cctggaatcc cagcactttt ggaagctgag gcaggcagat cacttgaggt taggatttca 11700
   agaccagcct ggctaacata gtaaagccct gtctctacta aaaatacaaa aattagccag 11760
   gtatggtggc gagcttctgt agccccagct actcaggaga ctgaggcagg agaatcactt 11820
   gaacccggga agtggggggg tgcagtgacc caagatcacg ccactgcatt ccagcctggg 11880
   caacagagca agactccatc tcaaaaaaaa aagttctatt tccttgaata aaattttccg 11940
   aagtttaaac tttaggaata aaactattaa acccgtattt actcatccag atacccaccc 12000
   cccttgttga gattctctcc caattatcaa aatgtgtagc atatttaact accaagagct 12060
   aaacatcatt aagactgaaa tgtattaaga aggatgtata ggccaggcac ggtgtctcac 12120
   gcctgtaatc ccaacacttt gggaggccaa gtcgggcgga tcacgaggtc aggagatgga 12180
   gaccatcctg gccaacatgg tgaaaccccc tctctactaa aaatacaaaa attagccagg 12240
   caggtggcag gcacctgtaa tcccagctac tccagaggct gaggcaggac aatcacttga 12300
   acctgggagg cagaggctgc agtgagctga ggttgtacca attgcactcc agcctaggta 12360
   acgagcaaca ctccatctca aaaaaagaaa aaaaaaaaga tgtataattt ggaactgtta 12420
   agaggcattt taaaga 12436
<210> 2
   <211> 429
   <212> PRT
   <213> Homo sapiens
<400> 2
<210> 3
   <211> 1290
   <212> DNA
   <213> Homo sapiens
<400> 3
   atgcagctga ggaatcccga gctccacctg ggctgtgctc tggctctgcg gttcctggcc 60
   ctcgtgtcct gggacatccc tggcgctagg gccctcgata acggactggc ccggaccccc 120
   acaatgggat ggctccactg ggaaaggttc atgtgcaatc tggactgtca ggaggaaccc 180
   gactcctgca tcagcgaaaa gctcttcatg gagatggccg agctgatggt gagcgagggc 240
   tggaaggacg ccggctacga gtatctgtgc atcgatgact gctggatggc ccctcaaagg      300
   gactccgaag gcaggctgca ggctgatccc caaaggtttc cccacggaat ccggcagctc      360
   gccaactacg tgcattccaa gggcctcaag ctcggcatct acgccgacgt gggcaacaaa      420
   acatgcgccg gattccccgg cagcttcggc tactacgaca tcgacgccca gacattcgct      480
   gattggggag tggacctgct gaagttcgac ggctgttact gcgattccct ggaaaacctg      540
   gccgacggct acaaacacat gtccctcgcc ctgaaccgga caggcaggtc catcgtgtac      600
   agctgcgagt ggcccctgta catgtggcct ttccagaagc ccaactacac agagatcagg      660
   cagtactgca accactggag gaacttcgct gacatcgacg actcctggaa gagcatcaag      720
   agcatcctgg actggaccag cttcaaccag gagaggatcg tggacgtggc tggacccgga      780
   ggctggaacg accccgatat gctggtgatt ggcaacttcg gactgagctg gaaccagcag      840
   gtgacccaga tggccctgtg ggccattatg gccgctcccc tgttcatgtc caacgacctg      900
   aggcacatca gcccccaggc caaggctctg ctgcaggaca aggatgtgat cgccatcaac      960
   caggaccccc tgggcaagca gggctaccag ctgaggcaag gagataactt cgaggtgtgg     1020
   gagaggcccc tgtccggact ggcttgggcc gtggccatga tcaatcggca ggagatcggc     1080
   ggaccccggt cctacaccat tgctgtggcc agcctgggaa aaggagtcgc ctgcaacccc     1140
   gcctgcttca ttacccagct gctccccgtg aagcggaagc tgggcttcta tgagtggacc     1200
   agcaggctga ggtcccatat caatcctacc ggcaccgtcc tcctccagct cgagaatacc     1260
   atgcagatga gcctcaagga tctgctgtga                                      1290

## Description

### CROSS-REFERENCE TO RELATED APPLICATIONS

This application claims priority to U.S. Provisional Application Serial No. 62/550,984 filed on August 28, 2017.

### TECHNICAL FIELD

The present invention relates generally to migalastat for use in the treatment of Fabry disease.

### BACKGROUND

Fabry disease is a progressive, X-linked inborn error of glycosphingolipid metabolism caused by a deficiency in the lysosomal enzyme α-galactosidase A (a-Gal A) as a result of mutations in the α-Gal A gene (GLA). Despite being an X-linked disorder, females can express varying degrees of clinical manifestations. Fabry is a rare disease with incidence estimated between 1 in 40,000 males to 1 in 117,000 in the general population. Moreover, there are variants of later onset phenotype of Fabry disease that can be under-diagnosed, as they do not present with classical signs and symptoms. This, and newborn screening for Fabry disease, suggests that the actual incidence of Fabry disease can be higher than currently estimated.

Untreated, life expectancy in Fabry patients is reduced and death usually occurs in the fourth or fifth decade because of vascular disease affecting the kidneys, heart and/or central nervous system. The enzyme deficiency leads to intracellular accumulation of the substrate, globotriaosylceramide (GL-3) in the vascular endothelium and visceral tissues throughout the body. Gradual deterioration of renal function and the development of azotemia, due to glycosphingolipid deposition, usually occur in the third to fifth decades of life, but can occur as early as in the second decade. Renal lesions are found in both hemizygous (male) and heterozygous (female) patients.

Cardiac disease as a result of Fabry disease occurs in most males and many females. Early cardiac findings include left ventricular enlargement, valvular involvement and conduction abnormalities. Mitral insufficiency is the most frequent valvular lesion typically present in childhood or adolescence. Cerebrovascular manifestations result primarily from multifocal small-vessel involvement and can include thromboses, transient ischemic attacks, basilar artery ischemia and aneurysm, seizures, hemiplegia, hemianesthesia, aphasia, labyrinthine disorders, or cerebral hemorrhages. Average age of onset of cerebrovascular manifestations is 33.8 years. Personality change and psychotic behavior can manifest with increasing age.

One approved therapy for treating Fabry disease is enzyme replacement therapy (ERT), which typically involves intravenous, infusion of a purified form of the corresponding wild-type protein. Two α-Gal A products are currently available for the treatment of Fabry disease: agalsidase alfa (Replagal^{®}, Shire Human Genetic Therapies) and agalsidase beta (Fabrazyme^{®}; Sanofi Genzyme Corporation). While ERT is effective in many settings, the treatment also has limitations. ERT has not been demonstrated to decrease the risk of stroke, cardiac muscle responds slowly, and GL-3 elimination from some of the cell types of the kidneys is limited. Some patients also develop immune reactions to ERT. Mehta A. et al., LANCET, 2009, vol. 374, p. 1986-1996 discloses enzyme replacement therapy (ERT) with agalsidase alfa to increase midwall fractional shortening (MWFS) in patients with Fabry disease.

Accordingly, there remains a need for therapies for the treatment of Fabry disease, especially for enhancing the cardiac function.

### SUMMARY

The present invention relates to a formulation comprising an effective amount of migalastat or salt thereof for use in increasing midwall fractional shortening (MWFS) in a patient having Fabry disease, wherein the effective amount is 100 mg to 150 mg free base equivalent (FBE) and the formulation is administered to the patient every other day.

Further embodiments of the invention are set out in the appended set of claims.

In one or more embodiments, the patient has impaired MWFS prior to initiating administration of the migalastat or salt thereof.

In one or more embodiments, the patient has left ventricular hypertrophy (LVH) prior to initiating administration of the migalastat or salt thereof.

In one or more embodiments, the migalastat or salt thereof enhances α-Gal A activity.

In one or more embodiments, the patient is administered about 123 mg FBE of the migalastat or salt thereof every other day.

In one or more embodiments, the patient is administered about 123 mg of migalastat free base every other day.

In one or more embodiments, the patient is administered about 150 mg of migalastat hydrochloride every other day.

In one or more embodiments, the formulation comprises an oral dosage form. In one or more embodiments, the oral dosage form comprises a tablet, a capsule or a solution.

In one or more embodiments, the migalastat or salt thereof is administered for at least 12 months.

In one or more embodiments, the migalastat or salt thereof is administered for at least 24 months.

In one or more embodiments, the patient is an ERT-naïve patient.

In one or more embodiments, the administration of migalastat or a salt thereof provides an average increase in MWFS in a group of ERT-naïve patients with impaired MWFS of at least about 1% after 24 months of administration of migalastat or a salt thereof.

In one or more embodiments, the patient is an ERT-experienced patient.

In one or more embodiments, the administration of migalastat or a salt thereof provides an average change in MWFS in a group of ERT-experienced patients with impaired MWFS of greater than about -0.5% after 30 months of administration of migalastat or a salt thereof.

### BRIEF DESCRIPTION OF THE DRAWINGS

Further features of the present invention will become apparent from the following written description and the accompanying figures, in which:
FIGS. 1A-E show the full DNA sequence of the human wild-type GLA gene (SEQ ID NO: 1);
FIG. 2 shows the wild-type α-Gal A protein (SEQ ID NO: 2); and
FIG. 3 shows the nucleic acid sequence encoding the wild-type α-Gal A protein (SEQ ID NO: 3).

### DETAILED DESCRIPTION

### Definitions

The terms used in this specification generally have their ordinary meanings in the art, within the context of this invention and in the specific context where each term is used. Certain terms are discussed below, or elsewhere in the specification, to provide additional guidance to the practitioner in describing the compositions and methods of the invention and how to make and use them.

The term "Fabry disease" refers to an X-linked inborn error of glycosphingolipid catabolism due to deficient lysosomal α-Gal A activity. This defect causes accumulation of the substrate globotriaosylceramide ("GL-3", also known as Gb3 or ceramide trihexoside) and related glycosphingolipids in vascular endothelial lysosomes of the heart, kidneys, skin, and other tissues. Another substrate of the enzyme is plasma globotriaosylsphingosine ("plasma lyso-Gb₃").

The term "atypical Fabry disease" refers to patients with primarily cardiac manifestations of the α-Gal A deficiency, namely progressive GL-3 accumulation in myocardial cells that leads to significant enlargement of the heart, particularly the left ventricle.

A "carrier" is a female who has one X chromosome with a defective α-Gal A gene and one X chromosome with the normal gene and in whom X chromosome inactivation of the normal allele is present in one or more cell types. A carrier is often diagnosed with Fabry disease.

A "patient" refers to a subject who has been diagnosed with or is suspected of having a particular disease. The patient may be human or animal.

A "Fabry patient" refers to an individual who has been diagnosed with or suspected of having Fabry disease and has a mutated α-Gal A as defined further below. Characteristic markers of Fabry disease can occur in male hemizygotes and female carriers with the same prevalence, although females typically are less severely affected.

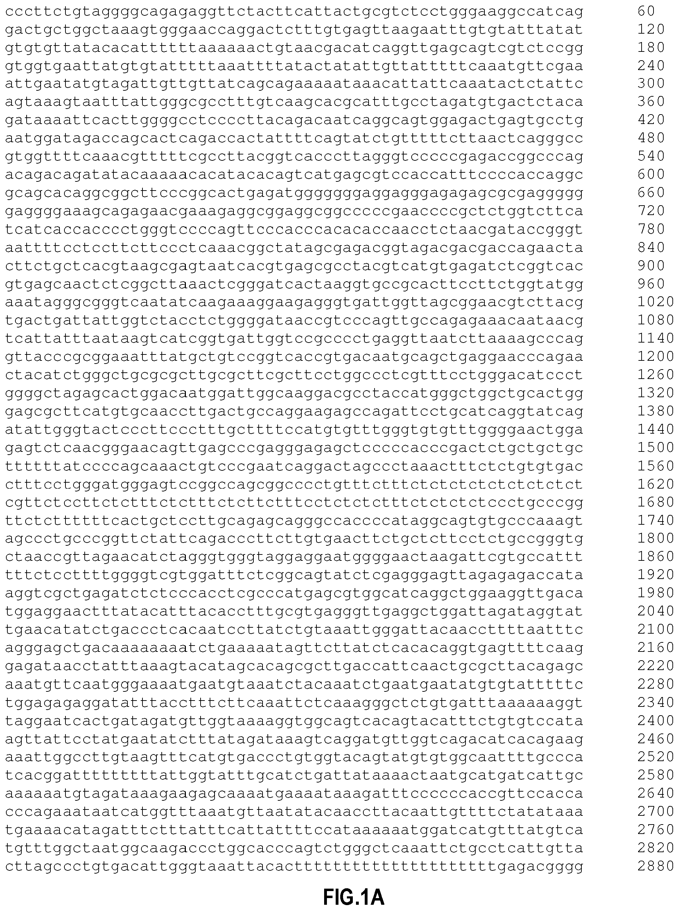

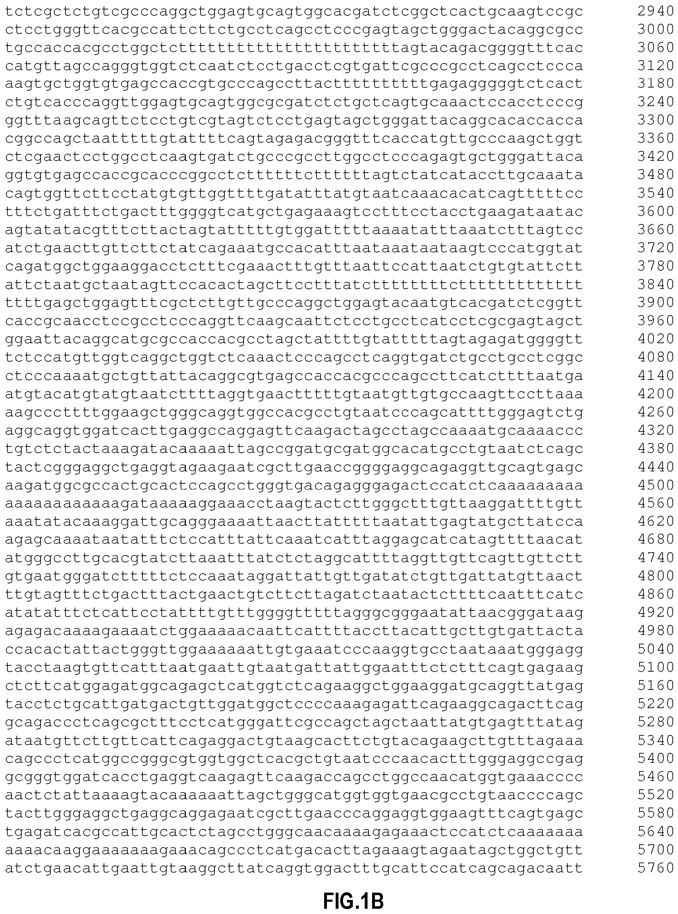

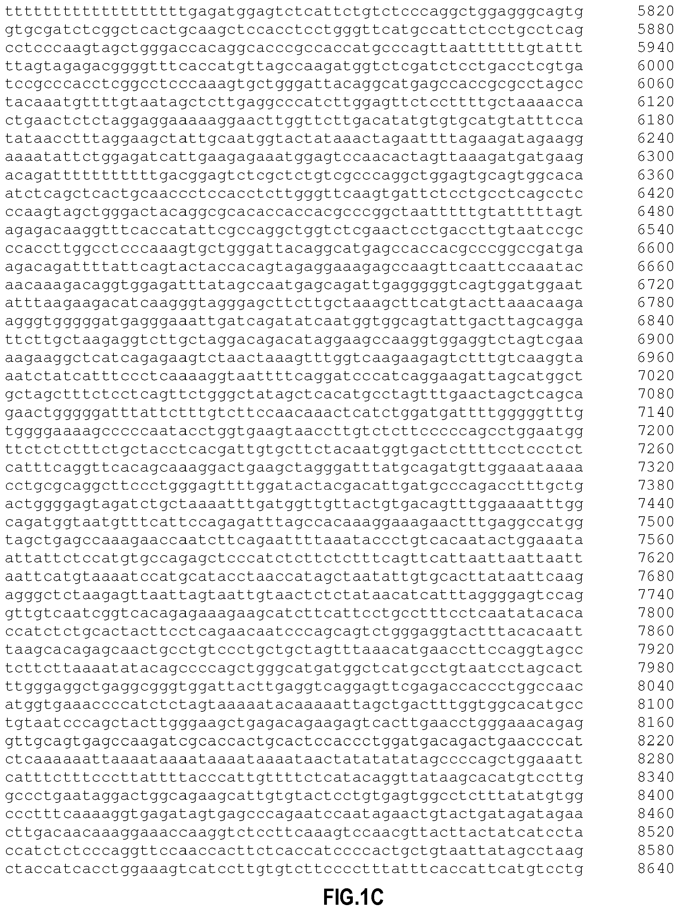

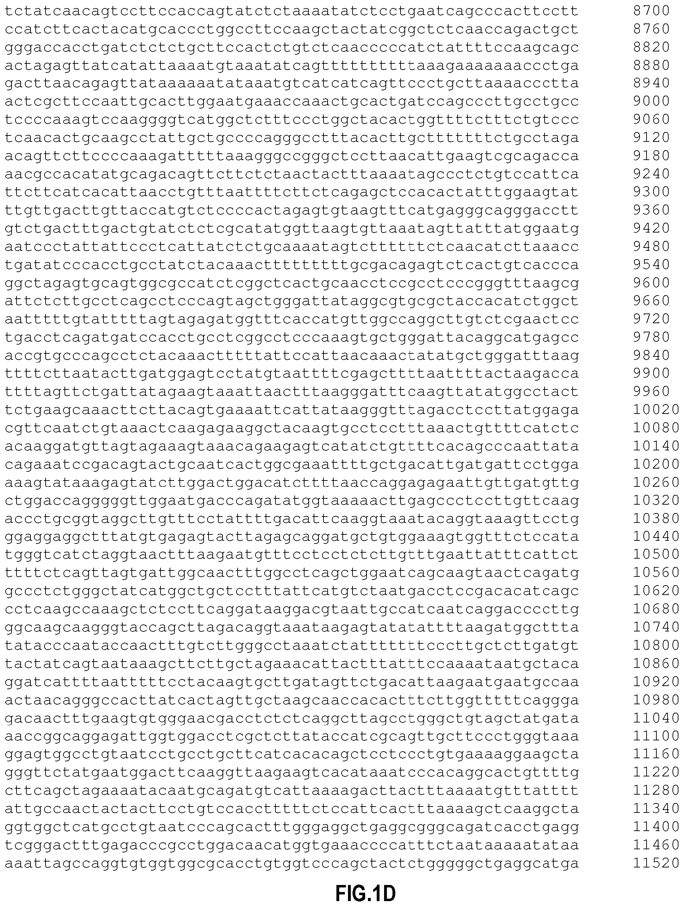

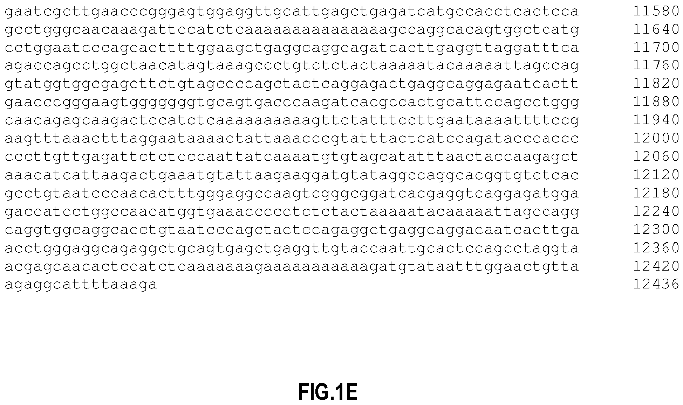

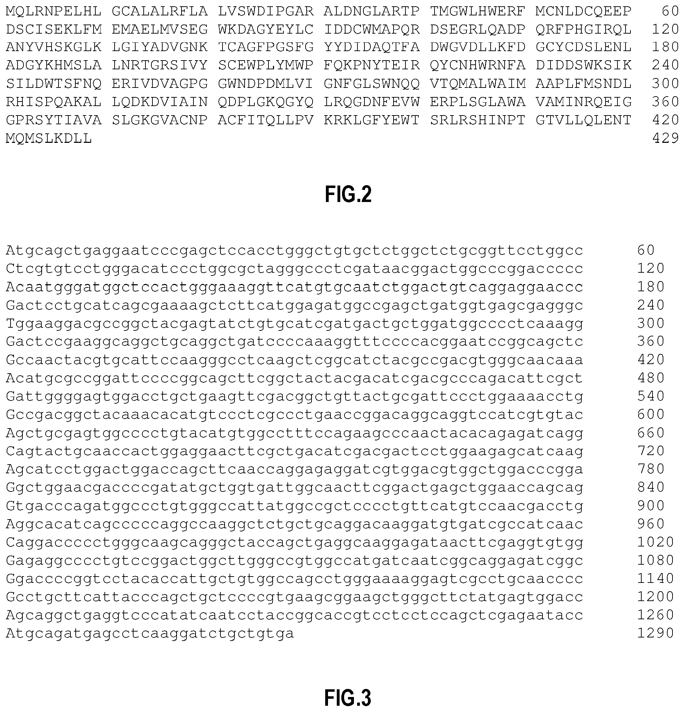

Human α-galactosidase A (a-Gal A) refers to an enzyme encoded by the human GLA gene. The full DNA sequence of α-Gal A, including introns and exons, is available in GenBank Accession No. X14448.1 and shown in FIG. 1A-E (SEQ ID NO: 1). The human α-Gal A enzyme consists of 429 amino acids and is available in GenBank Accession Nos. X14448.1 and U78027.1 and shown in FIG.2 (SEQ ID NO: 2). The nucleic acid sequence that only includes the coding regions (i.e. exons) of SEQ ID NO: 1 is shown in FIG. 3 (SEQ ID NO: 3).

The term "mutant protein" includes a protein which has a mutation in the gene encoding the protein which results in the inability of the protein to achieve a stable conformation under the conditions normally present in the endoplasmic reticulum (ER). The failure to achieve a stable conformation results in a substantial amount of the enzyme being degraded, rather than being transported to the lysosome. Such a mutation is sometimes called a "conformational mutant." Such mutations include, but are not limited to, missense mutations, and in-frame small deletions and insertions.

As used herein in one embodiment, the term "mutant α-Gal A" includes an α-Gal A which has a mutation in the gene encoding α-Gal A which results in the inability of the enzyme to achieve a stable conformation under the conditions normally present in the ER. The failure to achieve a stable conformation results in a substantial amount of the enzyme being degraded, rather than being transported to the lysosome.

As used herein, the term "pharmacological chaperone" ("PC") refers to any molecule including a small molecule, protein, peptide, nucleic acid, carbohydrate, etc. that specifically binds to a protein and has one or more of the following effects: (i) enhances the formation of a stable molecular conformation of the protein; (ii) induces trafficking of the protein from the ER to another cellular location, preferably a native cellular location, *i.e.*, prevents ER-associated degradation of the protein; (iii) prevents aggregation of misfolded proteins; and/or (iv) restores or enhances at least partial wild-type function and/or activity to the protein. A compound that specifically binds to *e.g.,* α-Gal A, means that it binds to and exerts a chaperone effect on the enzyme and not a generic group of related or unrelated enzymes. More specifically, this term does not refer to endogenous chaperones, such as BiP, or to non-specific agents which have demonstrated non-specific chaperone activity against various proteins, such as glycerol, DMSO or deuterated water, *i.e.*, chemical chaperones. The PC may be a reversible competitive inhibitor. The PC according to the invention is migalastat or a salt thereof. In another embodiment, the PC is migalastat free base *(e.g.,* 123 mg of migalastat free base). In yet another embodiment, the PC is a salt of migalastat *(e.g.,* 150 mg of migalastat HCl).

A "competitive inhibitor" of an enzyme can refer to a compound which structurally resembles the chemical structure and molecular geometry of the enzyme substrate to bind the enzyme in approximately the same location as the substrate. Thus, the inhibitor competes for the same active site as the substrate molecule, thus increasing the Km. Competitive inhibition is usually reversible if sufficient substrate molecules are available to displace the inhibitor, *i.e.*, competitive inhibitors can bind reversibly. Therefore, the amount of enzyme inhibition depends upon the inhibitor concentration, substrate concentration, and the relative affinities of the inhibitor and substrate for the active site.

As used herein, the term "specifically binds" refers to the interaction of a pharmacological chaperone with a protein such as α-Gal A, specifically, an interaction with amino acid residues of the protein that directly participate in contacting the pharmacological chaperone. A pharmacological chaperone specifically binds a target protein, *e.g.,* α-Gal A, to exert a chaperone effect on the protein and not a generic group of related or unrelated proteins. The amino acid residues of a protein that interact with any given pharmacological chaperone may or may not be within the protein's "active site." Specific binding can be evaluated through routine binding assays or through structural studies, e.g., co-crystallization, NMR, and the like. The active site for α-Gal A is the substrate binding site.

"Deficient α-Gal A activity" refers to α-Gal A activity in cells from a patient which is below the normal range as compared (using the same methods) to the activity in normal individuals not having or suspected of having Fabry or any other disease (especially a blood disease).

As used herein, the terms "enhance α-Gal A activity" or "increase α-Gal A activity" refer to increasing the amount of α-Gal A that adopts a stable conformation in a cell contacted with a pharmacological chaperone specific for the α-Gal A, relative to the amount in a cell (preferably of the same cell-type or the same cell, *e.g.,* at an earlier time) not contacted with the pharmacological chaperone specific for the α-Gal A. This term also refers to increasing the trafficking of α-Gal A to the lysosome in a cell contacted with a pharmacological chaperone specific for the α-Gal A, relative to the trafficking of α-Gal A not contacted with the pharmacological chaperone specific for the protein. These terms refer to both wild-type and mutant α-Gal A. In one embodiment, the increase in the amount of α-Gal A in the cell is measured by measuring the hydrolysis of an artificial substrate in lysates from cells that have been treated with the PC. An increase in hydrolysis is indicative of increased α-Gal A activity.

The term "a-Gal A activity" refers to the normal physiological function of a wild-type α-Gal A in a cell. For example, α-Gal A activity includes hydrolysis of GL-3.

A "responder" is an individual diagnosed with or suspected of having a lysosomal storage disorder (LSD), such, for example Fabry disease, whose cells exhibit sufficiently increased α-Gal A activity, respectively, and/or amelioration of symptoms or enhancement in surrogate markers, in response to contact with a PC. Non-limiting examples of enhancements in surrogate markers for Fabry are lyso-GB3 and those disclosed in US Patent Application Publication No. U.S. 2010/0113517.

Non-limiting examples of improvements in surrogate markers for Fabry disease disclosed in U.S. 2010/0113517 include increases in α-Gal A levels or activity in cells *(e.g.,* fibroblasts) and tissue; reductions in of GL-3 accumulation; decreased plasma concentrations of homocysteine and vascular cell adhesion molecule-1 (VCAM-1); decreased GL-3 accumulation within myocardial cells and valvular fibrocytes; reduction in plasma lyso-Gb₃; reduction in cardiac hypertrophy (especially of the left ventricle), amelioration of valvular insufficiency, and arrhythmias; amelioration of proteinuria; decreased urinary concentrations of lipids such as CTH, lactosylceramide, ceramide, and increased urinary concentrations of glucosylceramide and sphingomyelin; the absence of laminated inclusion bodies (Zebra bodies) in glomerular epithelial cells; improvements in renal function; mitigation of hypohidrosis; the absence of angiokeratomas; and improvements in hearing abnormalities such as high frequency sensorineural hearing loss progressive hearing loss, sudden deafness, or tinnitus. Improvements in neurological symptoms include prevention of transient ischemic attack (TIA) or stroke; and amelioration of neuropathic pain manifesting itself as acroparaesthesia (burning or tingling in extremities). Another type of clinical marker that can be assessed for Fabry disease is the prevalence of deleterious cardiovascular manifestations.

"Midwall fractional shortening" or "MWFS" is a measure of systolic function that identifies hypertensive patients who have evidence of target-organ damage, impaired contractile reserve, and increased mortality.

The term "cardiac function" refers to the performance of a patient's heart. For example, one assessment of cardiac function is left ventricular systolic function, which refers to the emptying characteristics of the left heart. Left ventricular systolic function can be evaluated in several ways, including, but not limited to, left ventricular ejection fraction (LVEF), endocardial fractional shortening (EFS) and MWFS.

As used herein, the phrase "stabilizing cardiac function" and similar terms refer to reducing or arresting the decline in cardiac function and/or restoring cardiac function. As untreated Fabry patients are expected to have significant decreases in cardiac function over time, enhancements in the rate of cardiac function deterioration and/or enhancements in cardiac function demonstrate a benefit of migalastat therapy as described herein. Stabilizing cardiac function includes stabilizing MWFS. "Stabilizing MWFS" likewise refers to reducing or arresting the decline in MWFS.

The term "enhancing cardiac function" refers to a beneficial change in at least one parameter used to evaluate cardiac function. If a patient's parameter is at the lower end of a normal range or is below the normal range for that parameter, than a beneficial change in that parameter is an increase in that parameter. For example, an increase in MWFS for a patient having a low MWFS is an enhancement in that parameter. Similarly, if a patient's parameter is at the upper end of a normal range or is above the normal range for that parameter, than a beneficial change in that parameter is a decrease in that parameter. In an aspect, "enhancing cardiac function" comprises one or more of (i) improving left ventricular function, (ii) improving fractional shortening, (iii) improving ejection fraction, (iv) reducing end-diastolic volume, and (v) normalizing of heart geometry.

As used herein, the term "impaired MWFS" refers to a patient having an MWFS below the normal range. The normal range of MWFS for a female is at least 15% and the normal range of MWFS for a male is at least 14%. Thus, impaired MWFS for a female patient is <15% and impaired MWFS for a male patient is <14%.

As used herein, the term "normalizing MWFS" refers to increasing the MWFS of a patient from an impaired MWFS to within the normal range. Thus, normalizing MWFS for a female patient is increasing MWFS from <15% to at least 15%, and normalizing MWFS for a male patient is increasing MWFS from <14% to at least 14%.

As used herein, the term "left ventricular hypertrophy" or "LVH" refers to a patient having a left ventricular mass index (LVMi) above the normal range of 43-95 g/m² for females and 49-115 g/m² for males. Thus, LVH refers to a LVMi > 95 g/m² for females or > 115 g/m² for males.

The dose that achieves one or more of the aforementioned responses is a "therapeutically effective dose."

The phrase "pharmaceutically acceptable" refers to molecular entities and compositions that are physiologically tolerable and do not typically produce untoward reactions when administered to a human. In some embodiments, as used herein, the term "pharmaceutically acceptable" means approved by a regulatory agency of the Federal or a state government or listed in the U.S. Pharmacopoeia or other generally recognized pharmacopoeia for use in animals, and more particularly in humans. The term "carrier" in reference to a pharmaceutical carrier refers to a diluent, adjuvant, excipient, or vehicle with which the compound is administered. Such pharmaceutical carriers can be sterile liquids, such as water and oils. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin, 18th Edition, or other editions.

As used herein, the term "isolated" means that the referenced material is removed from the environment in which it is normally found. Thus, an isolated biological material can be free of cellular components, *i.e.,* components of the cells in which the material is found or produced. In the case of nucleic acid molecules, an isolated nucleic acid includes a PCR product, an mRNA band on a gel, a cDNA, or a restriction fragment. In another embodiment, an isolated nucleic acid is preferably excised from the chromosome in which it may be found, and more preferably is no longer joined to non-regulatory, non-coding regions, or to other genes, located upstream or downstream of the gene contained by the isolated nucleic acid molecule when found in the chromosome. In yet another embodiment, the isolated nucleic acid lacks one or more introns. Isolated nucleic acids include sequences inserted into plasmids, cosmids, artificial chromosomes, and the like. Thus, in a specific embodiment, a recombinant nucleic acid is an isolated nucleic acid. An isolated protein may be associated with other proteins or nucleic acids, or both, with which it associates in the cell, or with cellular membranes if it is a membrane-associated protein. An isolated organelle, cell, or tissue is removed from the anatomical site in which it is found in an organism. An isolated material may be, but need not be, purified.

The term "enzyme replacement therapy" or "ERT" refers to the introduction of a non-native, purified enzyme into an individual having a deficiency in such enzyme. The administered protein can be obtained from natural sources or by recombinant expression (as described in greater detail below). The term also refers to the introduction of a purified enzyme in an individual otherwise requiring or benefiting from administration of a purified enzyme, e.g., suffering from enzyme insufficiency. The introduced enzyme may be a purified, recombinant enzyme produced *in vitro,* or protein purified from isolated tissue or fluid, such as, *e.g.,* placenta or animal milk, or from plants.

The term "ERT-naïve patient" refers to a Fabry patient that has never received ERT or has not received ERT for at least 6 months prior to initiating migalastat therapy.

The term "ERT-experienced patient" refers to a Fabry patient that was receiving ERT immediately prior to initiating migalastat therapy. In some embodiments, the ERT-experienced patient has received at least 12 months of ERT immediately prior to initiating migalastat therapy.

As used herein, the term "free base equivalent" or "FBE" refers to the amount of migalastat present in the migalastat or salt thereof. In other words, the term "FBE" means either an amount of migalastat free base, or the equivalent amount of migalastat free base that is provided by a salt of migalastat. For example, due to the weight of the hydrochloride salt, 150 mg of migalastat hydrochloride only provides as much migalastat as 123 mg of the free base form of migalastat. Other salts are expected to have different conversion factors, depending on the molecular weight of the salt.

The term "migalastat" encompasses migalastat free base or a pharmaceutically acceptable salt thereof (e.g., migalastat HCl), unless specifically indicated to the contrary.

The terms "mutation" and "variant" (e.g., as in "amenable mutation or variant") refer to a change in the nucleotide sequence of a gene or a chromosome. The two terms referred herein are typically used together - *e.g.,* as in "mutation or variant"- referring to the change in nucleotide sequence stated in the previous sentence. If only one of the two terms is recited for some reason, the missing term was intended to be included and one should understand as such. Furthermore, the terms "amenable mutation" and "amenable variant" refer to a mutation or variant that is amenable to PC therapy, *e.g.,* a mutation that is amenable to migalastat therapy. A particular type of amenable mutation or variant is a "HEK assay amenable mutation or variant", which is a mutation or variant that is determined to be amenable to migalastat therapy according to the criteria in the *in vitro* HEK assay described herein and in U.S. Patent No. 8,592,362.

The terms "about" and "approximately" shall generally mean an acceptable degree of error for the quantity measured given the nature or precision of the measurements. Typical, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 10- or 5-fold, and more preferably within 2-fold of a given value. Numerical quantities given herein are approximate unless stated otherwise, meaning that the term "about" or "approximately" can be inferred when not expressly stated.

### Fabry Disease

Fabry disease is a rare, progressive and devastating X-linked LSD. Mutations in the GLA gene result in a deficiency of the lysosomal enzyme, α-Gal A, which is required for glycosphingolipid metabolism. Beginning early in life, the reduction in α-Gal A activity results in an accumulation of glycosphingolipids, including GL-3 and plasma lyso-Gb3, and leads to the symptoms and life-limiting sequelae of Fabry disease, including pain, gastrointestinal symptoms, renal failure, cardiomyopathy, cerebrovascular events, and early mortality. Early initiation of therapy and lifelong treatment provide an opportunity to slow disease progression and prolong life expectancy.

Fabry disease encompasses a spectrum of disease severity and age of onset, although it has traditionally been divided into 2 main phenotypes, "classic" and "late-onset". The classic phenotype has been ascribed primarily to males with undetectable to low α-Gal A activity and earlier onset of renal, cardiac and/or cerebrovascular manifestations. The late-onset phenotype has been ascribed primarily to males with higher residual α-Gal A activity and later onset of these disease manifestations. Heterozygous female carriers typically express the late-onset phenotype but depending on the pattern of X-chromosome inactivation may also display the classic phenotype.

More than 1,000 Fabry disease-causing GLA mutations have been identified. Approximately 60% are missense mutations, resulting in single amino acid substitutions in the α-Gal A enzyme. Missense GLA mutations often result in the production of abnormally folded and unstable forms of α-Gal A and the majority are associated with the classic phenotype. Normal cellular quality control mechanisms in the ER block the transit of these abnormal proteins to lysosomes and target them for premature degradation and elimination. Many missense mutant forms are targets for migalastat, an α-Gal A-specific pharmacological chaperone.

The clinical manifestations of Fabry disease span a broad spectrum of severity and roughly correlate with a patient's residual α-Gal A levels. The majority of currently treated patients are referred to as classic Fabry patients, most of whom are males. These patients experience disease of various organs, including the kidneys, heart and brain, with disease symptoms first appearing in adolescence and typically progressing in severity until death in the fourth or fifth decade of life. A number of recent studies suggest that there are a large number of undiagnosed males and females that have a range of Fabry disease symptoms, such as impaired cardiac or renal function and strokes, that usually first appear in adulthood. Individuals with this type of Fabry disease, referred to as later-onset Fabry disease, tend to have higher residual α-Gal A levels than classic Fabry patients. Individuals with later-onset Fabry disease typically first experience disease symptoms in adulthood, and often have disease symptoms focused on a single organ, such as enlargement of the left ventricle or progressive kidney failure. In addition, later-onset Fabry disease may also present in the form of strokes of unknown cause.

Fabry patients have progressive kidney impairment, and untreated patients exhibit end-stage renal impairment by the fifth decade of life. Deficiency in α-Gal A activity leads to accumulation of GL-3 and related glycosphingolipids in many cell types including cells in the kidney. GL-3 accumulates in podocytes, epithelial cells and the tubular cells of the distal tubule and loop of Henle. Impairment in kidney function can manifest as proteinuria and reduced glomerular filtration rate.

Because Fabry disease is rare, involves multiple organs, has a wide age range of onset, and is heterogeneous, proper diagnosis is a challenge. Awareness is low among health care professionals and misdiagnoses are frequent. Diagnosis of Fabry disease is most often confirmed on the basis of decreased α-Gal A activity in plasma or peripheral leukocytes (WBCs) once a patient is symptomatic, coupled with mutational analysis. In females, diagnosis is even more challenging since the enzymatic identification of carrier females is less reliable due to random X-chromosomal inactivation in some cells of carriers. For example, some obligate carriers (daughters of classically affected males) have α-Gal A enzyme activities ranging from normal to very low activities. Since carriers can have normal α-Gal A enzyme activity in leukocytes, only the identification of an α-Gal A mutation by genetic testing provides precise carrier identification and/or diagnosis.

In one or more embodiments, mutant forms of α-Gal A are considered to be amenable to migalastat are defined as showing a relative increase (+10 µM migalastat) of >1.20-fold and an absolute increase (+ 10 µM migalastat) of ≥ 3.0% wild-type (WT) when the mutant form of α-Gal A is expressed in HEK-293 cells (referred to as the "HEK assay") according to Good Laboratory Practice (GLP)-validated *in vitro* assay (GLP HEK or Migalastat Amenability Assay). Such mutations are also referred to herein as "HEK assay amenable" mutations.

Previous screening methods have been provided that assess enzyme enhancement prior to the initiation of treatment. For example, an assay using HEK-293 cells has been utilized in clinical trials to predict whether a given mutation will be responsive to pharmacological chaperone (e.g., migalastat) treatment. In this assay, cDNA constructs are created. The corresponding α-Gal A mutant forms are transiently expressed in HEK-293 cells. Cells are then incubated ± migalastat (17 nM to 1 mM) for 4 to 5 days. After, α-Gal A levels are measured in cell lysates using a synthetic fluorogenic substrate (4-MU-α-Gal) or by western blot. This has been done for known disease-causing missense or small in-frame insertion/deletion mutations. Mutations that have previously been identified as responsive to a PC (e.g., migalastat) using these methods are listed in U.S. Patent No. 8,592,362.

### Pharmacological Chaperones

The binding of small molecule inhibitors of enzymes associated with LSDs can increase the stability of both mutant enzyme and the corresponding wild-type enzyme (see U.S. Pat. Nos. 6,274,597; 6,583,158; 6,589,964; 6,599,919; 6,916,829, and 7,141,582). In particular, administration of small molecule derivatives of glucose and galactose, which are specific, selective competitive inhibitors for several target lysosomal enzymes, effectively increased the stability of the enzymes in cells *in vitro* and, thus, increased trafficking of the enzymes to the lysosome. Thus, by increasing the amount of enzyme in the lysosome, hydrolysis of the enzyme substrates is expected to increase. The original theory behind this strategy was as follows: since the mutant enzyme protein is unstable in the ER (Ishii et al., Biochem. Biophys. Res. Comm. 1996; 220: 812-815), the enzyme protein is retarded in the normal transport pathway (ER→Golgi apparatus→endosomes→lysosome) and prematurely degraded. Therefore, a compound which binds to and increases the stability of a mutant enzyme, may serve as a "chaperone" for the enzyme and increase the amount that can exit the ER and move to the lysosomes. In addition, because the folding and trafficking of some wild-type proteins is incomplete, with up to 70% of some wild-type proteins being degraded in some instances prior to reaching their final cellular location, the chaperones can be used to stabilize wild-type enzymes and increase the amount of enzyme which can exit the ER and be trafficked to lysosomes.

The pharmacological chaperone according to the invention is migalastat or a salt thereof. The compound migalastat, also known as 1-deoxygalactonojirimycin (1-DGJ) or (2R,3S,4R,5S)-2-(hydroxymethyl) piperdine-3,4,5-triol is a compound having the following chemical formula:

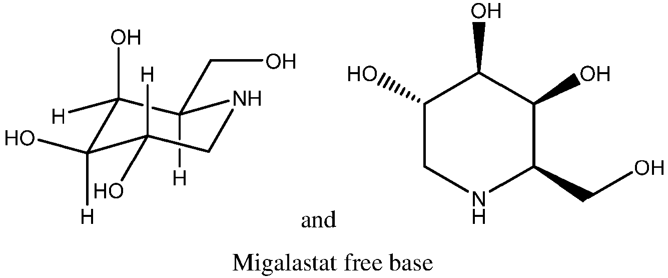

As discussed herein, pharmaceutically acceptable salts of migalastat may also be used in the present invention. When a salt of migalastat is used, the dosage of the salt will be adjusted so that the dose of migalastat received by the patient is equivalent to the amount which would have been received had the migalastat free base been used. One example of a pharmaceutically acceptable salt of migalastat is migalastat HCl:

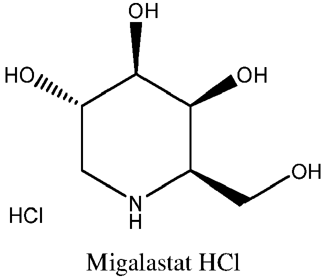

Migalastat is a low molecular weight iminosugar and is an analogue of the terminal galactose of GL-3. *In vitro* and *in vivo* pharmacologic studies have demonstrated that migalastat acts as a pharmacological chaperone, selectively and reversibly binding, with high affinity, to the active site of wild-type α-Gal A and specific mutant forms of α-Gal A, the genotypes of which are referred to as HEK assay amenable mutations. Migalastat binding stabilizes these mutant forms of α-Gal A in the endoplasmic reticulum facilitating their proper trafficking to lysosomes where dissociation of migalastat allows α-Gal A to reduce the level of GL-3 and other substrates. Approximately 30-50% of patients with Fabry disease have HEK assay amenable mutations; the majority of which are associated with the classic phenotype of the disease.

HEK assay amenable mutations include at least those mutations listed in a pharmacological reference table *(e.g.,* the ones recited in the U.S. or International Product labels for a migalastat product such as GALAFOLD^{®}). As used herein, "pharmacological reference table" refers to any publicly accessible written or electronic record, included in either the product label within the packaging of a migalastat product (e.g., GALAFOLD^{®}) or in a website accessible by health care providers, that conveys whether a particular mutation or variant is responsive to migalastat (e.g., GALAFOLD^{®}) PC therapy, and is not necessarily limited to written records presented in tabular form. A "pharmacological reference table" thus refers to any depository of information that includes one or more amenable mutations or variants. An exemplary pharmacological reference table for HEK assay amenable mutations can be found in the summary of product characteristics and/or prescribing information for GALAFOLD^{®} in various countries in which GALAFOLD^{®} is approved for use, or at a website such as www.galafoldamenabilitytable.com or www.fabrygenevariantsearch.com.

An exemplary pharmacological reference table for HEK assay amenable mutations is provided in Table 1 below. In one or more embodiments, if a double mutation is present on the same chromosome (males and females), that patient is considered HEK assay amenable if the double mutation is present in one entry in Table 1 (e.g., D55V/Q57L). In some embodiments, if a double mutation is present on different chromosomes (only in females) that patient is considered HEK assay amenable if either one of the individual mutations is present in Table 1.

**Table 1**

| **Nucleotide change** | **Nucleotide change** | **Protein sequence change** |
|---|---|---|
| c.7C>G | c.C7G | L3V |
| c.8T>C | c.T8C | L3P |
| c.[11G>T; 620A>C] | c.G11T/A620C | R4M/Y207S |
| c.37G>A | c.G37A | A13T |
| c.37G>C | c.G37C | A13P |
| c.43G>A | c.G43A | A15T |
| c.44C>G | c.C44G | A15G |
| c.53T>G | c.T53G | F18C |
| c.58G>C | c.G58C | A20P |
| c.59C>A | c.C59A | A20D |
| c.70T>C or c.70T>A | c.T70C or c.T70A | W24R |
| c.70T>G | c.T70G | W24G |
| c.72G>C or c.72G>T | c.G72C or c.G72T | W24C |
| c.95T>C | c.T95C | L32P |
| c.97G>C | c.G97C | D33H |
| c.97G>T | c.G97T | D33Y |
| c.98A>G | c.A98G | D33G |
| c.100A>G | c.A100G | N34D |
| c.101A>C | c.A101C | N34T |
| c,101A>G | c.A101G | N34S |
| c.102T>G or c.102T>A | c.T102G or c.T102A | N34K |
| c.103G>C or c.103G>A | c.G103C or c.G103A | G35R |
| c.104G>A | c.G104A | G35E |
| c.104G>C | c.G104C | G35A |
| c.104G>T | c.G104T | G35V |
| c.107T>C | c.T107C | L36S |
| c.107T>G | c.T107G | L36W |
| c.108G>C or c.108G>T | c.G108C or c.G108T | L36F |
| c.109G>A | c.G109A | A37T |
| c.110C>T | c.C110T | A37V |
| c.122C>T | c.C122T | T41I |
| c.124A>C or c.124A>T | c.A124C or c.A124T | M42L |
| c.124A>G | c.A124G | M42V |
| c.125T>A | c.T125A | M42K |
| c.125T>C | c.T125C | M42T |
| c.125T>G | c.T125G | M42R |
| c.126G>A or c.126G>C or c.126G>T | c.G126A or c.G126C or c.G126T | M42I |
| c.137A>C | c.A137C | H46P |
| c.142G>C | c.G142C | E48Q |
| c.152T>A | c.T152A | M51K |
| c.153G>A or c.153G>T or c.153G>C | c.G153A or c.G153T or c.G153C | M51I |
| c.157A>G | c.A157G | N53D |
| c.[157A>C; 158A>T] | c.A157C/A158T | N53L |
| c.160C>T | c.C160T | L54F |
| c.161T>C | c.T161C | L54P |
| c.164A>G | c.A164G | D55G |
| c.164A>T | c.A164T | D55V |
| c.[164A>T; 170A>T] | c.A164T/A170T | D55V/Q57L |
| c.167G>T | c.G167T | C56F |
| c.167G>A | c.G167A | C56Y |
| c.170A>T | c.A170T | Q57L |
| c.175G>A | c.G175A | E59K |
| c.178C>A | c.C178A | P60T |
| c.178C>T | c.C178T | P60S |
| c.179C>T | c.C179T | P60L |
| c.196G>A | c.G196A | E66K |
| c.197A>G | c.A197G | E66G |
| c.207C>A or c.207C>G | c.C207A or c.C207G | F69L |
| c.214A>G | c.A214G | M72V |
| c.216G>A or c.216G>T or c.216G>C | c.G216A or c.G216T or c.G216C | M72I |
| c.218C>T | c.C218T | A73V |
| c.227T>C | c.T227C | M76T |
| c.239G>A | c.G239A | G80D |
| c.247G>A | c.G247A | D83N |
| c.253G>A | c.G253A | G85S |
| c.254G>A | c.G254A | G85D |
| c.[253G>A; 254G>A] | c.G253A/G254A | G85N |
| c.[253G>A; 254G>T; 255T>G] | c.G253A/G254T/T255G | G85M |
| c.261G>C or c.261G>T | c.G261C or c.G261T | E87D |
| c.263A>C | c.A263C | Y88S |
| c.265C>T | c.C265T | L89F |
| c.272T>C | c.T272C | I91T |
| c.288G>A or c.288G>T or c.288G>C | c.G288A or c.G288T or c.G288C | M96I |
| c.289G>C | c.G289C | A97P |
| c.290C>T | c.C290T | A97V |
| c.305C>T | c.C305T | S102L |
| c.311G>T | c.G311T | G104V |
| c.316C>T | c.C316T | L106F |
| c.322G>A | c.G322A | A108T |
| c.326A>G | c.A326G | D109G |
| c.334C>G | c.C334G | R112G |
| c.335G>A | c.G335A | R112H |
| c.337T>A | c.T337A | F113I |
| c.337T>C or c.339T>A or c.339T>G | c.T337C or c.T339A or c.T339G | F113L |
| c.352C>T | c.C352T | R118C |
| c.361G>A | c.G361A | A121T |
| c.368A>G | c.A368G | Y123C |
| c.373C>T | c.C373T | H125Y |
| c.374A>T | c.A374T | H125L |
| c.376A>G | c.A376G | S126G |
| c.383G>A | c.G383A | G128E |
| c.399T>G | c.T399G | I133M |
| c.404C>T | c.C404T | A135V |
| c.408T>A or c.408T>G | c.T408A or c.T408G | D136E |
| c.416A>G | c.A416G | N139S |
| c.419A>C | c.A419C | K140T |
| c.427G>A | c.G427A | A143T |
| c.431G>A | c.G431A | G144D |
| c.431G>T | c.G431T | G144V |
| c.434T>C | c.T434C | F145S |
| c.436C>T | c.C436T | P146S |
| c.437C>G | c.C437G | P146R |
| c.454T>C | c.T454C | Y152H |
| c.455A>G | c.A455G | Y152C |
| c.466G>A | c.G466A | A156T |
| c.467C>T | c.C467T | A156V |
| c.471G>C or c.471G>T | c.G471C or c.G471T | Q157H |
| c.484T>G | c.T484G | W162G |
| c.493G>C | c.G493C | D165H |
| c.494A>G | c.A494G | D165G |
| c.[496C>G; 497T>G] | c.C496G/T497G | L166G |
| c.496C>G | c.C496G | L166V |
| c.496_497delinsTC | c.496_497delinsTC | L166S |
| c.499C>G | c.C499G | L167V |
| c.506T>C | c.T506C | F169S |
| c.511G>A | c.G511A | G171S |
| c.520T>C | c.T520C | C174R |
| c.520T>G | c.T520G | C174G |
| c.525C>G or c.525C>A | c.C525G or c.C525A | D175E |
| c.539T>G | c.T539G | L180W |
| c.540G>C | c.G540C | L180F |
| c.548G>C | c.G548C | G183A |
| c.548G>A | c.G548A | G183D |
| c.550T>A | c.T550A | Y184N |
| c.551A>G | c.A551G | Y184C |
| c.553A>G | c.A553G | K185E |
| c.559A>G | c.A559G | M187V |
| c.559_564dup | c.559_564dup | p.M187_S188dup |
| c.560T>C | c.T560C | M187T |
| c.561G>T or c.561G>A or c.561G>C | c.G561T or c.G561A or c.G561C | M187I |
| c.572T>A | c.T572A | L191Q |
| c.580A>G | c.A580G | T194A |
| c.581C>T | c.C581T | T194I |
| c.584G>T | c.G584T | G195V |
| c.586A>G | c.A586G | R196G |
| c.593T>C | c.T593C | I198T |
| c.595G>A | c.G595A | V199M |
| c.596T>C | c.T596C | V199A |
| c.596T>G | c.T596G | V199G |
| c.599A>G | c.A599G | Y200C |
| c.602C>T | c.C602T | S201F |
| c.602C>A | c.C602A | S201Y |
| c.608A>T | c.A608T | E203V |
| c.609G>C or c.609G>T | c.G609C or c.G609T | E203D |
| c.610T>G | c.T610G | W204G |
| c.613C>A | c.C613A | P205T |
| c.613C>T | c.C613T | P205S |
| c.614C>T | c.C614T | P205L |
| c.619T>C | c.T619C | Y207H |
| c.620A>C | c.A620C | Y207S |
| c.623T>G | c.T623G | M208R |
| c.628C>T | c.C628T | P210S |
| c.629C>T | c.C629T | P210L |
| c.638A>G | c.A638G | K213R |
| c.638A>T | c.A638T | K213M |
| c.640C>T | c.C640T | P214S |
| c.641C>T | c.C641T | P214L |
| c.643A>G | c.A643G | N215D |
| c.644A>G | c.A644G | N215S |
| c.644A>T | c.A644T | N215I |
| c.[644A>G; 937G>T] | c.A644G/G937T | N215S/D313Y |
| c.646T>G | c.T646G | Y216D |
| c.647A>C | c.A647C | Y216S |
| c.647A>G | c.A647G | Y216C |
| c.655A>C | c.A655C | I219L |
| c.656T>A | c.T656A | I219N |
| c.656T>C | c.T656C | I219T |
| c.659G>A | c.G659A | R220Q |
| c.659G>C | c.G659C | R220P |
| c.662A>C | c.A662C | Q221P |
| c.671A>C | c.A671C | N224T |
| c.671A>G | c.A671G | N224S |
| c.673C>G | c.C673G | H225D |
| c.683A>G | c.A683G | N228S |
| c.687T>A or c.687T>G | c.T687A or c.T687G | F229L |
| c.695T>C | c.T695C | I232T |
| c.713G>A | c.G713A | S238N |
| c.716T>C | c.T716C | I239T |
| c.720G>C or c.720G>T | c.G720C or c.G720T | K240N |
| c.724A>G | c.A724G | I242V |
| c.724A>T | c.A724T | I242F |
| c.725T>A | c.T725A | I242N |
| c.725T>C | c.T725C | I242T |
| c.728T>G | c.T728G | L243W |
| c.729G>C or c.729G>T | c.G729C or c.G729T | L243F |
| c.730G>A | c.G730A | D244N |
| c.730G>C | c.G730C | D244H |
| c.733T>G | c.T733G | W245G |
| c.740C>G | c.C740G | S247C |
| c.747C>G or c.747C>A | c.C747G or c.C747A | N249K |
| c.748C>A | c.C748A | Q250K |
| c.749A>C | c.A749C | Q250P |
| c.749A>G | c.A749G | Q250R |
| c.750G>C | c.G750C | Q250H |
| c.758T>C | c.T758C | I253T |
| c.758T>G | c.T758G | I253S |
| c.760-762delGTT | c.760_762delGTT | p.V254del |
| c.769G>C | c.G769C | A257P |
| c.770C>G | c.C770G | A257G |
| c.772G>C or c.772G>A | c.G772C or c.G772A | G258R |
| c.773G>T | c.G773T | G258V |
| c.776C>G | c.C776G | P259R |
| c.776C>T | c.C776T | P259L |
| c.779G>A | c.G779A | G260E |
| c.779G>C | c.G779C | G260A |
| c.781G>A | c.G781A | G261S |
| c.781G>C | c.G781C | G261R |
| c.781G>T | c.G781T | G261C |
| c.788A>G | c.A788G | N263S |
| c.790G>T | c.G790T | D264Y |
| c.794C>T | c.C794T | P265L |
| c.800T>C | c.T800C | M267T |
| c.805G>A | c.G805A | V269M |
| c.806T>C | c.T806C | V269A |
| c.809T>C | c.T809C | I270T |
| c.810T>G | c.T810G | I270M |
| c.811G>A | c.G811A | G271S |
| c.[811G>A; 937G>T] | c.G811A/G937T | G271S/D313Y |
| c.812G>A | c.G812A | G271D |
| c.823C>G | c.C823G | L275V |
| c.827G>A | c.G827A | S276N |
| c.829T>G | c.T829G | W277G |
| c.831G>T or c.831G>C | c.G831T or c.G831C | W277C |
| c.832A>T | c.A832T | N278Y |
| c.835C>G | c.C835G | Q279E |
| c.838C>A | c.C838A | Q280K |
| c.840A>T or c.840A>C | c.A840T or c.A840C | Q280H |
| c.844A>G | c.A844G | T282A |
| c.845C>T | c.C845T | T282I |
| c.850A>G | c.A850G | M284V |
| c.851T>C | c.T851C | M284T |
| c.860G>T | c.G860T | W287L |
| c.862G>C | c.G862C | A288P |
| c.866T>G | c.T866G | I289S |
| c.868A>C or c.868A>T | c.A868C or c.A868T | M290L |
| c.869T>C | c.T869C | M290T |
| c.870G>A or c.870G>C or c.870G>T | c.G870A or c.G870C or c.G870T | M290I |
| c.871G>A | c.G871A | A291T |
| c.877C>A | c.C877A | P293T |
| c.881T>C | c.T881C | L294S |
| c.884T>G | c.T884G | F295C |
| c.886A>G | c.A886G | M296V |
| c.886A>T or c.886A>C | c.A886T or c.A886C | M296L |
| c.887T>C | c.T887C | M296T |
| c.888G>A or c.888G>T or c.888G>C | c.G888A or c.G888T or c.G888C | M296I |
| c.893A>G | c.A893G | N298S |
| c.897C>G or c.897C>A | c.C897G or c.C897A | D299E |
| c.898C>T | c.C898T | L300F |
| c.899T>C | c.T899C | L300P |
| c.901C>G | c.C901G | R301G |
| c.902G>C | c.G902C | R301P |
| c.902G>A | c.G902A | R301Q |
| c.902G>T | c.G902T | R301L |
| c.907A>T | c.A907T | I303F |
| c.908T>A | c.T908A | I303N |
| c.911G>A | c.G911A | S304N |
| c.911G>C | c.G911C | S304T |
| c.919G>A | c.G919A | A307T |
| c.922A>G | c.A922G | K308E |
| c.924A>T or c.924A>C | c.A924T or c.A924C | K308N |
| c.925G>C | c.G925C | A309P |
| c.926C>T | c.C926T | A309V |
| c.928C>T | c.C928T | L310F |
| c.931C>G | c.C931G | L311V |
| c.935A>G | c.A935G | Q312R |
| c.936G>T or c.936G>C | c.G936T or c.G936C | Q312H |
| c.937G>T | c.G937T | D313Y |
| c.[937G>T; 1232G>A] | c.G937T/G1232A | D313Y/G411D |
| c.938A>G | c.A938G | D313G |
| c.946G>A | c.G946A | V316I |
| c.947T>G | c.T947G | V316G |
| c.950T>C | c.T950C | I317T |
| c.955A>T | c.A955T | I319F |
| c.956T>C | c.T956C | I319T |
| c.959A>T | c.A959T | N320I |
| c.962A>G | c.A962G | Q321R |
| c.962A>T | c.A962T | Q321L |
| c.963G>C or c.963G>T | c.G963C or c.G963T | Q321H |
| c.964G>A | c.G964A | D322N |
| c.964G>C | c.G964C | D322H |
| c.966C>A or c.966C>G | c.C966A or c.C966G | D322E |
| c.968C>G | c.C968G | P323R |
| c.973G>A | c.G973A | G325S |
| c.973G>C | c.G973C | G325R |
| c.978G>C or c.978G>T | c.G978C or c.G978T | K326N |
| c.979C>G | c.C979G | Q327E |
| c.980A>T | c.A980T | Q327L |
| c.983G>C | c.G983C | G328A |
| c.989A>G | c.A989G | Q330R |
| c.1001G>A | c.G1001A | G334E |
| c.1010T>C | c.T1010C | F337S |
| c.1012G>A | c.G1012A | E338K |
| c.1016T>A | c.T1016A | V339E |
| c.1027C>A | c.C1027A | P343T |
| c.1028C>T | c.C1028T | P343L |
| c.1033T>C | c.T1033C | S345P |
| c.1046G>C | c.G1046C | W349S |
| c.1055C>G | c.C1055G | A352G |
| c.1055C>T | c.C1055T | A352V |
| c.1061T>A | c.T1061A | I354K |
| c.1066C>G | c.C1066G | R356G |
| c.1066C>T | c.C1066T | R356W |
| c.1067G>A | c.G1067A | R356Q |
| c.1067G>C | c.G1067C | R356P |
| c.1072G>C | c.G1072C | E358Q |
| c.1073A>C | c.A1073C | E358A |
| c.1073A>G | c.A1073G | E358G |
| c.1074G>T or c.1074G>C | c.G1074T or c.G1074C | E358D |
| c.1076T>C | c.T1076C | I359T |
| c.1078G>A | c.G1078A | G360S |
| c.1078G>T | c.G1078T | G360C |
| c.1079G>A | c.G1079A | G360D |
| c.1082G>A | c.G1082A | G361E |
| c.1082G>C | c.G1082C | G361A |
| c.1084C>A | c.C1084A | P362T |
| c.1085C>T | c.C1085T | P362L |
| c.1087C>T | c.C1087T | R363C |
| c.1088G>A | c.G1088A | R363H |
| c.1102G>A | c.G1102A | A368T |
| c.1117G>A | c.G1117A | G373S |
| c.1124G>A | c.G1124A | G375E |
| c.1153A>G | c.A1153G | T385A |
| c.1168G>A | c.G1168A | V390M |
| c.1172A>C | c.A1172C | K391T |
| c.1175G>C | c.G1175C | R392T |
| c.1184G>A | c.G1184A | G395E |
| c.1184G>C | c.G1184C | G395A |
| c.1192G>A | c.G1192A | E398K |
| c.1202_1203insGACTTC | c.1202_1203insGACTTC | p.T400_S401dup |
| c.1208T>C | c.T1208C | L403S |
| c.1225C>G | c.C1225G | P409A |
| c.1225C>T | c.C1225T | P409S |
| c.1225C>A | c.C1225A | P409T |
| c.1228A>G | c.A1228G | T410A |
| c.1229C>T | c.C1229T | T410I |
| c.1232G>A | c.G1232A | G411D |
| c.1235C>A | c.C1235A | T412N |
| c.1253A>G | c.A1253G | E418G |
| c.1261A>G | c.A1261G | M421V |

| | | |
|---|---|---|
| Dosing, Formulation and Administration | | |

According to the invention , the Fabry patient is administered migalastat or salt thereof at a frequency of once every other day (also referred to as "QOD"). In various embodiments, the doses described herein pertain to migalastat hydrochloride or an equivalent dose of migalastat or a salt thereof other than the hydrochloride salt. In some embodiments, these doses pertain to the free base of migalastat. In alternate embodiments, these doses pertain to a salt of migalastat. In further embodiments, the salt of migalastat is migalastat hydrochloride. The administration of migalastat or a salt of migalastat is referred to herein as "migalastat therapy".

The effective amount of migalastat or salt thereof can be in the range from about 100 mg FBE to about 150 mg FBE. Exemplary doses include about 100 mg FBE, about 105 mg FBE, about 110 mg FBE, about 115 mg FBE, about 120 mg FBE, about 123 mg FBE, about 125 mg FBE, about 130 mg FBE, about 135 mg FBE, about 140 mg FBE, about 145 mg FBE or about 150 mg FBE.

Again, it is noted that 150 mg of migalastat hydrochloride is equivalent to 123 mg of the free base form of migalastat. Thus, in one or more embodiments, the dose is 150 mg of migalastat hydrochloride or an equivalent dose of migalastat or a salt thereof other than the hydrochloride salt, administered at a frequency of once every other day. As set forth above, this dose is referred to as 123 mg FBE of migalastat. In further embodiments, the dose is 150 mg of migalastat hydrochloride administered at a frequency of once every other day. In other embodiments, the dose is 123 mg of the migalastat free base administered at a frequency of once every other day.

In various embodiments, the effective amount is about 122 mg, about 128 mg, about 134 mg, about 140 mg, about 146 mg, about 150 mg, about 152 mg, about 159 mg, about 165 mg, about 171 mg, about 177 mg or about 183 mg of migalastat hydrochloride.

Accordingly, in various embodiments, migalastat therapy includes administering 123 mg FBE at a frequency of once every other day, such as 150 mg of migalastat hydrochloride every other day.

The administration of migalastat or salt thereof may be for a certain period of time. In one or more embodiments, the migalastat or salt thereof is administered for a duration of at least 28 days, such as at least 30, 60 or 90 days or at least 3, 4, 5, 6, 7, 8, 9, 10, 11, 12, 16, 20, 24, 30 or 36 months or at least 1, 2, 3, 4 or 5 years. In various embodiments, the migalastat therapy is long-term migalastat therapy of at least 6 months, such as at least 6, 7, 8, 9, 10, 11, 12, 16, 20, 24, 30 or 36 months or at least 1, 2, 3, 4 or 5 years.

Administration of migalastat or salt thereof according to the present invention may be in a formulation suitable for any route of administration, but is preferably administered in an oral dosage form such as a tablet, capsule or solution. As one example, the patient is orally administered capsules each containing 150 mg migalastat hydrochloride or an equivalent dose of migalastat or a salt thereof other than the hydrochloride salt.

In some embodiments, migalastat or a salt thereof is administered orally. In one or more embodiments, migalastat or a salt thereof is administered by injection. Migalastat or a salt thereof may be accompanied by a pharmaceutically acceptable carrier, which may depend on the method of administration.

In one or more embodiments, migalastat or a salt thereof is administered as monotherapy, and can be in a form suitable for any route of administration, including e.g., orally in the form tablets or capsules or liquid, or in sterile aqueous solution for injection. In other embodiments, migalastat or a salt thereof is provided in a dry lyophilized powder to be added to the formulation of the replacement enzyme during or immediately after reconstitution to prevent enzyme aggregation *in vitro* prior to administration.

When migalastat or a salt thereof is formulated for oral administration, the tablets or capsules can be prepared by conventional means with pharmaceutically acceptable excipients such as binding agents (e.g., pregelatinized maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulfate). The tablets may be coated by methods well known in the art. Liquid preparations for oral administration may take the form of, for example, solutions, syrups or suspensions, or they may be presented as a dry product for constitution with water or another suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p- hydroxybenzoates or sorbic acid). The preparations may also contain buffer salts, flavoring, coloring and sweetening agents as appropriate. Preparations for oral administration may be suitably formulated to give controlled release of the active chaperone compound.

The pharmaceutical formulations of migalastat or a salt thereof suitable for parenteral/injectable use generally include sterile aqueous solutions (where water soluble), or dispersions and sterile powders for the extemporaneous preparation of sterile injectable solutions or dispersion. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be brought about by various antibacterial and antifungal agents, for example, parabens, chlorobutanol, phenol, benzyl alcohol, sorbic acid, and the like. In many cases, it will be reasonable to include isotonic agents, for example, sugars or sodium chloride. Prolonged absorption of the injectable compositions can be brought about by the use in the compositions of agents delaying absorption, for example, aluminum monosterate and gelatin.

Sterile injectable solutions are prepared by incorporating the purified enzyme (if any) and migalastat or a salt thereof in the required amount in the appropriate solvent with various of the other ingredients enumerated above, as required, followed by filter or terminal sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The formulation can contain an excipient. Pharmaceutically acceptable excipients which may be included in the formulation are buffers such as citrate buffer, phosphate buffer, acetate buffer, bicarbonate buffer, amino acids, urea, alcohols, ascorbic acid, and phospholipids; proteins, such as serum albumin, collagen, and gelatin; salts such as EDTA or EGTA, and sodium chloride; liposomes; polyvinylpyrollidone; sugars, such as dextran, mannitol, sorbitol, and glycerol; propylene glycol and polyethylene glycol *(e.g.,* PEG-4000, PEG-6000); glycerol; glycine or other amino acids; and lipids. Buffer systems for use with the formulations include citrate; acetate; bicarbonate; and phosphate buffers. Phosphate buffer is a preferred embodiment.

The route of administration of the chaperone compound may be oral or parenteral, including intravenous, subcutaneous, intra-arterial, intraperitoneal, ophthalmic, intramuscular, buccal, rectal, vaginal, intraorbital, intracerebral, intradermal, intracranial, intraspinal, intraventricular, intrathecal, intracisternal, intracapsular, intrapulmonary, intranasal, transmucosal, transdermal, or via inhalation.

Administration of the above-described parenteral formulations of the chaperone compound may be by periodic injections of a bolus of the preparation, or may be administered by intravenous or intraperitoneal administration from a reservoir which is external (e.g., an i.v. bag) or internal *(e.g.,* a bioerodable implant).

In one or more embodiments, migalastat or a salt thereof is administered in combination with ERT. ERT increases the amount of protein by exogenously introducing wild-type or biologically functional enzyme by way of infusion. This therapy has been developed for many genetic disorders, including LSDs such as Fabry disease, as referenced above. After the infusion, the exogenous enzyme is expected to be taken up by tissues through non-specific or receptor-specific mechanism. In general, the uptake efficiency is not high, and the circulation time of the exogenous protein is short. In addition, the exogenous protein is unstable and subject to rapid intracellular degradation as well as having the potential for adverse immunological reactions with subsequent treatments. In one or more embodiments, the chaperone is administered at the same time as replacement enzyme *(e.g.,* replacement α-Gal A). In some embodiments, the chaperone is co-formulated with the replacement enzyme (e.g., replacement α-Gal A).

In one or more embodiments, a patient is switched from ERT to migalastat therapy. In some embodiments, a patient on ERT is identified, the patient's ERT is discontinued, and the patient begins receiving migalastat therapy. The migalastat therapy can be in accordance with any of the methods described herein.

### Cardiac Function

The dosing regimens described herein can stabilize and/or enhance cardiac function *(e.g.,* left ventricular systolic function) in Fabry patients. As untreated Fabry patients typically exhibit a deterioration of cardiac function over time, both enhancements in and maintenance of cardiac function are indications of a benefit of migalastat therapy. As described in further detail in the Examples below, Phase 3 studies have found that migalastat therapy increases and/or stabilizes MWFS in both ERT-experienced and ERT-naïve patients. These Phase 3 studies also found that migalastat therapy normalizes MWFS in some patients with impaired MWFS. Accordingly, migalastat therapy can be used to treat Fabry patients by stabilizing MWFS, increasing MWFS and/or normalizing MWFS in ERT-naïve and/or ERT-experienced Fabry patients, including patients with impaired MWFS.

The migalastat therapy may arrest or decrease the reduction in MWFS and/or increase MWFS for a Fabry patient compared to the same patient without treatment with migalastat therapy. In one or more embodiments, the migalastat therapy provides a change in MWFS for a patient that is greater than (i.e., more positive than) -2%, such as greater than or equal to about -1.5%, -1.4%, -1.3%, -1.2%, -1.1%, -1%, -0.9%, -08.%, -0.7%, -0.6%, -0.5%, - 0.4%, -0.3%, -0.2%, -0.1%, 0%, 0.1%, 0.2%, 0.3%, 0.4%, 0.5%, 0.6%, 0.7%, 0.8%, 0.9%, 1%, 1.1%, 1.2%, 1.3%, 1.4%, 1.5%, 1.6%, 1.7%, 1.8%, 1.9%, 2%, 2.1%, 2.2%, 2.3%, 2.4% or 2.5%. In one or more embodiments, the Fabry patient is an ERT-experienced patient. In one or more embodiments, the Fabry patient is an ERT-naïve patient. In one or more embodiments, the Fabry patient has impaired MWFS prior to initiating migalastat therapy.

In one or more embodiments, the migalastat therapy provides an average change of MWFS in a group of ERT-naïve patients of at least about 0% after 12 months of administration of migalastat or a salt thereof. In various embodiments, the average increase in the group of ERT-naïve patients after 12 months of administration of migalastat or a salt thereof is at least about 0.05%, about 0.1%, about 0.15% or about 0.2%. In one or more embodiments, the ERT-naïve patients have impaired MWFS prior to initiating migalastat therapy.

In one or more embodiments, the migalastat therapy provides an average change of MWFS in a group of ERT-naïve patients of at least about 0% after 24 months of administration of migalastat or a salt thereof. In various embodiments, the average increase in the group of ERT-naïve patients after 12 months of administration of migalastat or a salt thereof is at least about 0.05%, about 0.1%, about 0.15%, about 0.2%, about 0.3%, about 0.4%, about 0.5%, about 0.6%, about 0.7%, about 0.8%, about 0.9%, about 1%, about 1.1%, about 1.2%, about 1.3%, about 1.4% or about 1.5%. In one or more embodiments, the ERT-naïve patients have impaired MWFS prior to initiating migalastat therapy.

In one or more embodiments, the migalastat therapy provides an average change of MWFS in a group of ERT-naïve patients of at least about 0% after 36 months of administration of migalastat or a salt thereof. In various embodiments, the average increase in the group of ERT-naïve patients after 12 months of administration of migalastat or a salt thereof is at least about 0.05%, about 0.1%, about 0.15%, about 0.2%, about 0.3%, about 0.4%, about 0.5%, about 0.6%, about 0.7%, about 0.8%, about 0.9%, about 1%, about 1.1%, about 1.2%, about 1.3%, about 1.4% or about 1.5%. In one or more embodiments, the ERT-naïve patients have impaired MWFS prior to initiating migalastat therapy.

In one or more embodiments, the migalastat therapy provides an average change of MWFS in a group of ERT-naïve patients of at least about 0% after 48 months of administration of migalastat or a salt thereof. In various embodiments, the average increase in the group of ERT-naïve patients after 12 months of administration of migalastat or a salt thereof is at least about 0.05%, about 0.1%, about 0.15%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9% about 2%, about 2.1%, about 2.2%, about 2.3%, about 2.4% or about 2.5%. In one or more embodiments, the ERT-naïve patients have impaired MWFS prior to initiating migalastat therapy.

In one or more embodiments, the migalastat therapy provides an average change of MWFS in a group of ERT-naïve patients of greater about -1.5% after 30 months of administration of migalastat or a salt thereof. In various embodiments, the average increase in the group of ERT-naïve patients after 12 months of administration of migalastat or a salt thereof is greater than -1.5%, -1.4%, -1.3%, -1.2%, -1.1%, -1%, -0.9%, -08.%, -0.7%, -0.6%, - 0.5%, -0.4%, -0.3%, -0.2%, -0.1% or 0%. In one or more embodiments, the ERT-experienced patients have impaired MWFS prior to initiating migalastat therapy.

### EXAMPLES

### EXAMPLE 1: Dosing Regimens for the Treatment of ERT-Naïve Fabry Patients Using Migalastat Hydrochloride

This example describes a Phase 3 study of migalastat therapy in ERT-naïve Fabry patients.

***Patient Enrollment.*** Eligible patients were 16-74 years old and had genetically-confirmed Fabry disease; had either never received or had not received ERT for ≥6 months; had a GLA mutation that resulted in a mutant protein that would respond to migalastat, based on the human embryonic kidney-293 (HEK) assay used at the time of enrollment; had an eGFR >30 ml/minute/1.73m², and had a urinary GL-3 ≥4 times the upper limit of normal.

***Study Design.*** Following eligibility-baseline assessments (2 months), patients were randomized to Stage 1-- 6 months of double-blind administration of 150 mg migalastat hydrochloride or placebo every other day. All patients completing Stage 1 were eligible to receive open-label migalastat in Stage 2 (months 6-12) and for an additional year (months 13-24) thereafter. The primary objective was to compare the effect of migalastat to placebo on kidney GL-3 as assessed by histological scoring of the number of inclusions in interstitial capillaries after 6 months of treatment. The secondary objectives of Stage 1 were to compare the effect of migalastat to placebo on urine GL-3 levels, on renal function, 24-hours urinary protein, and on safety and tolerability. The tertiary objectives were cardiac function, patient-reported outcomes, exploratory kidney analyses, and white blood cell α-Gal A activity. Study completers were eligible to enroll in the open-label extension study for up to 5 years.

***Kidney Histology Assessment.*** Each patient underwent a baseline kidney biopsy, as well as repeat kidney biopsies at 6 and 12 months. The number of GL-3 inclusions per kidney interstitial capillary per patient at baseline, and at 6 and 12 months was quantitatively assessed in 300 capillaries by 3 independent pathologists blinded to treatment and visit. All values for each individual biopsy at a given time were averaged prior to statistical analysis.

GL-3 changes in podocytes, endothelial cells, and mesangial cells, and glomerular sclerosis, were assessed qualitatively by the same 3 pathologists blinded to treatment/visit.

***Globotriaosylceramide and Globotriaosylsphingosine.*** Plasma lyso-Gb3 and 24-hour urine GL-3 were analyzed by liquid chromatography-mass-spectroscopy using a novel stable isotope-labeled internal standard, 13C6-lyso-Gb3 (lower-limit-of-quantification: 0.200 ng/mL, 0.254 nmol/L).

***Renal Function Assessment.*** Annualized rates of change (mL/min/1.73m²/year) were calculated using Chronic Kidney Disease Epidemiology Collaboration-eGFR_{CKD-EPI}) and measured iohexol clearance-mGFRᵢₒₕₑₓₒₗ).

***Echocardiography.*** LVMi, left posterior wall thickness, diastolic, interventricular septum thickness, diastolic and other parameters were assessed through blinded, centralized evaluation.

***Patient-Reported Outcomes.*** Patient-reported outcomes were assessed using the Gastrointestinal-Symptoms-Rating-Scale (GSRS), Short Form-36v2TM and Brief-Pain-Inventory-Pain-Severity-Component.

***Safety Analysis and Adverse Events.*** Randomized patients receiving ≥1 dose were included in the safety analysis, which comprised vital signs, physical exams, electrocardiograms, clinical labs, and adverse events.

***Statistical Analyses for Kidney Interstitial Capillary GL-3 Substrate.*** The primary Stage 1 (6 month) endpoint (ITT population with baseline biopsies, n=64) was the proportion of patients in the migalastat and placebo groups with a ≥50% reduction in GL-3 inclusions per interstitial capillary. Two other Stage 1 endpoints were assessed (modified-ITT population: randomized patients with paired baseline and month 6 biopsies; n=60): percent change in GL-3 inclusions per interstitial capillary, and percent of interstitial capillaries with zero GL-3 inclusions.

Efficacy analyses for GL-3 inclusions per interstitial capillary and other prespecified endpoints in Stage 2 (months 6-12) and the open-label-extension (months 12-24) were based on the modified intention to treat (mITT) - population consisting of randomized patients with mutant α-Gal A enzyme shown to be suitable for migalastat treatment by the validated assay; n=50).

### Results

**Baseline Characteristics.** Sixty-seven patients (16-74 years-old; 64% female) with potentially responsive mutant α-Gal A were randomized (ITT population). Table 2 provides the baseline characteristics for the 50 patients in the ITT population with suitable mutant α-Gal A. There were no statistically significant differences in baseline parameters.

**Table 2: Baseline Characteristics**

| **Parameter** | | **Treatment Group** | | **Total** (N=50) |
|---|---|---|---|---|
| | | **Migalastat HCl** (N=28) | **Placebo to Migalastat HCl** (N=22) | |
| **Age (year) (n)** | | 28 | 22 | 50 |
| | Mean±SD | 41.5±13 | 45.1±8.0 | 43.1±11 |
| | Median | 37.0 | 45.5 | 45.0 |
| **Weight (kg) (n)** | | 28 | 22 | 50 |
| | Mean±SD | 72.6±15.35 | 76.1±16.52 | 74.1±15.81 |
| | Median | 72.3 | 74.0 | 72.8 |
| | **Number of Years of Diagnosis of Fabrv Disease (n)** | 28 | 21 | 49 |
| | Mean±SD | 5.6±6.89 | 7.3±8.80 | 6.3±7.73 |
| | Median | 4.1 | 4.1 | 4.1 |
| **Number of patients previously on ERT (>6 months prior to baseline)** (%) | | 4 (14.3%) | 7 (31.8%) | 11 (22.0%) |
| **Use of ACEi/ARB/Ri at Baseline** | | | | |
| | Yes (%) | 9 (32.1%) | 12 (54.5%) | 21 (42.0%) |
| | No (%) | 19 (67.9%) | 10 (45.5%) | 29 (58.0%) |
| **Proteinuria >150mg/24h (%)** | | 17 (60.7%) | 18 (81.8%) | 35 (70.0%) |
| **Proteinuria >300mg/24h (%)** | | 8 (28.6%) | 11 (50.0%) | 19 (38.0%) |
| **Proteinuria >1000mg/24h (%)** | | 3 (10.7%) | 3 (13.6%) | 6 (12.0%) |
| **mGFR _{Iohexol} (mL/min/1.73m²) (n)** | | 27 | 21 | 48 |
| | Mean±SD | 79.95±30.9 | 83.12±22.8 | 81.34±27.5 |
| | Median | 84.90 | 82.20 | 83.40 |
| **eGFR_{CKD-EPI} (mL/min/1.73m²)** | | 28 | 22 | 50 |
| | Mean±SD | 94.4±27.0 | 90.6±17.1 | 92.7±23.0 |
| | Median | 96.6 | 93.5 | 94.0 |
| **Lyso-Gb₃ (n)** | | 18 | 13 | 31 |
| | Mean (nmol/L) ±SD | 47.3±62 | 41.9±39 | 45.0±53 |

Published reports of clinical phenotype(s) associated with the genotypes of patients with suitable mutations (n=50) indicate that 30 (60%) had mutations associated with the classic phenotype of Fabry disease, one (2%) with the non-classic phenotype, three (6%) with both phenotypes, and 16 (32%) not yet classified. Residual WBC α-Gal A activity <3% was found in 14 of 16 (87%) males; 29 of 31 (94%) males and females had elevated plasma lyso-Gb3, and 47 of 50 (94%) males and females had multi-organ system disease.

***Baseline MWFS.*** At baseline, impaired MWFS (<15% for females and <14% for males) was reported in 9 patients.

**Migalastat and Cardiac Function.** This study of ERT-naïve patients found that migalastat therapy increased MWFS in patients with impaired MWFS at baseline. Table 3 below shows the change from baseline in MWFS after migalastat therapy.

**Table 3: Percent Changes From Baseline in MWFS Over Time with Migalastat Therapy in Patients With Impaired MWFS at Baseline (Patients with Amenable Mutations)**

| | **Baseline Mean MWFS = 11.3%** **Timepoint** | | | | |
|---|---|---|---|---|---|
| | **Month 12** | **Month 24** | **Month 36** | **Month 48** | **LOCF** |
| **n** | 7 | 8 | 4 | 3 | 8 |
| **Mean change from baseline** | 0.1% | 1.4% | 1.4% | 2.4% | 1.9% |
| **95% CI** | -1.2%, -1.4% | -1.3%, 4.0% | -1.5%, 4.3% | -2.1%, 6.9% | -0.8%, 4.5% |
| **Any increase** | 2/7 (29%) | 5/8 (63%) | 3/4 (75%) | 3/3 (100%) | 6/8 (75%) |
| **Normalization** | 0 | 2/8 (25%) | 2/4 (50%) | 2/3 (67%) | 3/8 (38%) |

| | | | | | |
|---|---|---|---|---|---|
| Last observation carried forward (LOCF) analyses are based on last study assessment including any unscheduled or early termination visits; Abnormal MWFS is <15% for females and <14% for males. | | | | | |

As can be seen from Table 3, LOCF analysis of ERT-naïve patients with impaired MWFS at baseline showed mean changes in MWFS of 1.9% (95% CI -0.8%, 4.5%; n=8) over 48 months of migalastat therapy. 6/8 (75%) of patients had an increase of MWFS after migalastat therapy, with 3/8 (38%) demonstrating normalization of MWFS.

MWFS was also analyzed in patients with LVH at baseline. The change from baseline in MWFS in patients with LVH at baseline is provided in Table 4 below:

**Table 4: Change From Baseline in MWFS With Migalastat in Patients With LVH at Baseline (Patients with Amenable Mutations)**

| | **Baseline** | **Change from Baseline** | | | | |
|---|---|---|---|---|---|---|
| | | **Month 12** | **Month 24** | **Month 36** | **Month 48** | **LOCF** |
| **n** | 10 | 9 | 9 | 5 | 4 | 10 |
| **%, mean (SD) or (95% CI)** | 12.2 (2.6) | 0.2 (-0.8, 1.1) | 0.9 (-1.6,3.4) | 0.7 (-2.1,3.4) | 0.6 (-5.7, 6.9) | 1.0 (-1.5,3.5) |
| **Any increase** | - | 4/9 (44%) | 5/9 (56%) | 3/5 (60%) | 3/4 (75%) | 7/10 (70%) |
| **Normalization** | - | 2/9 (22%) | 2/9 (22%) | 1/5 (20%) | 0 | 2/10 (20%) |

| | | | | | | |
|---|---|---|---|---|---|---|
| LOCF analyses are based on last study assessment including any unscheduled or early termination visits; LVH subgroup: LVMi >95 g/m² (females) or >115 g/m2 (males). | | | | | | |

As can be seen from Table 4, LOCF analysis of ERT-naïve patients with LVH at baseline showed mean changes in MWFS of 1.0% (95% CI -1.5%, 3.5%; n=10) over 48 months of migalastat therapy. 7/10 (70%) of patients had an increase of MWFS after migalastat therapy, with 2/10 (20%) demonstrating normalization of MWFS.

**Safety and Adverse Events.** During Stage 1, the treatment-emergent adverse events were similar between groups. Adverse events with a higher frequency in patients receiving migalastat compared to placebo were headache (12/34 patients-35% versus 7/33 patients-21%) and nasopharyngitis (6/34 patients-18% versus 2/34-6%). The most frequently reported adverse events for Stage 2 were headache (9/63 patients-14%) and procedural pain (7/63 patients-11%-related to kidney biopsies) and, for the open-label-extension, proteinuria (9/57 patients-16%), headache (6/57 patients-11%), and bronchitis (6/57 patients-11%). Most adverse events were mild or moderate in severity. No adverse events led to migalastat discontinuation.

Six patients experienced serious adverse events during Stage 1 (2: migalastat; 4: placebo), 5 during Stage 2, and 11 during the open-label-extension. Two serious adverse events were assessed as possibly related to migalastat by the investigator-- fatigue and paresthesia. Both occurred in the same patient between months 12-24 and resolved. No individual serious adverse event was reported by >1 patient. Two patients discontinued migalastat due to serious adverse events; both were deemed unrelated to migalastat. No deaths were reported.

Treatment-emergent proteinuria was reported in 9 patients (16%) between months 12-24, and in one case, was judged as migalastat-related. In 5 patients, the 24-month values were in the same range as baseline. Three patients with suitable mutations had overt baseline proteinuria (>1g/24-hr), which increased over 24 months. In 23/28 patients with baseline proteinuria <300 mg/24-h, 24-hour urine protein remained stable during migalastat treatment.

There was no progression to end-stage renal disease, no cardiac death and no stroke as defined in Banikazemi et al.. There was a single case of transient ischemic attack-judged unrelated to migalastat.

Analyses of vital sign, physical findings, laboratory, and ECG parameters did not reveal any clinically relevant effect of migalastat.

### EXAMPLE 2: Dosing Regimens for the Treatment of ERT-Experienced Fabry Patients Using Migalastat Hydrochloride

This example describes a Phase 3 study of migalastat therapy in ERT-experienced Fabry patients.

***Patient Enrollment.*** Eligible patients were 16-74 years old and had genetically-confirmed Fabry disease; had received ERT for ≥12 months; had a GLA mutation that resulted in a mutant protein that would respond to migalastat, based on the human embryonic kidney-293 (HEK) assay used at the time of enrollment; had an eGFR ≥ 30 ml/minute/1.73m²; and had an ERT dose level and regimen that had been stable for at least 3 months.

***Study Design.*** Following eligibility-baseline assessments, 57 patients were randomized to 18 months of migalastat therapy or ERT, followed by followed by 12 months of migalastat therapy. The migalastat dosing regimen was 150 mg of migalastat hydrochloride every other day. The primary objective was to compare the effect of migalastat to ERT on renal function assessed by mGFRᵢₒₕₑₓₒₗ after 18 months of treatment. The secondary objectives were to compare the effect of migalastat to ERT on: renal function (assessed by eGFR and 24-hour urine protein); composite clinical outcome (assessed by time to occurrence of renal, cardiac, cerebrovascular events or death); cardiac function (assessed by echocardiography) and patient reported outcomes (pain and quality of life).

***Baseline MWFS.*** At baseline, impaired MWFS (<15% for females and <14% for males) was reported in 19 (14 migalastat, 5 ERT) patients.

### Results

**Migalastat and Cardiac Function.** This study of ERT-experienced patients found that migalastat therapy stabilized MWFS in patients with impaired MWFS at baseline. LOCF analysis of patients with impaired MWFS at baseline showed mean changes in baseline of -0.2% (95% CI -1.3%, 1.0%; n=14) over 30 months of migalastat therapy. LOCF analysis of patients with impaired MWFS at baseline showed mean changes in MWFS of -0.6% (95% CI -2.6%, 1.4%; n=5) over 18 months of treatment with ERT.

The embodiments described herein are intended to be illustrative of the present formulation for use and are not intended to limit the scope of the present invention. The appended claims should not be limited by the specific embodiments set forth in the examples, but should be given the broadest interpretation consistent with the description as a whole.

## Claims

1. A formulation comprising an effective amount of migalastat or salt thereof for use in increasing midwall fractional shortening (MWFS) in a patient having Fabry disease, wherein the effective amount is 100 mg to 150 mg free base equivalent (FBE ) and the formulation is administered to the patient every other day.

2. The formulation for use of claim 1, wherein the patient has impaired MWFS prior to initiating administration of the migalastat or salt thereof.

3. The formulation for use of claim 1 or 2, wherein the migalastat or salt thereof enhances α-galactosidase A activity.

4. The formulation for use of any one of claims 1-3, wherein effective amount is 123 mg FBE .

5. The formulation for use of any one of claims 1-4, wherein the effective amount is 123 mg of migalastat free base.

6. The formulation for use of any one of claims 1-4, wherein the effective amount is 150 mg of migalastat hydrochloride.

7. The formulation for use of any one of claims 1-6, wherein the formulation comprises an oral dosage form.

8. formulation for use of claim 7, wherein the oral dosage form comprises a tablet, a capsule or a solution.

9. The formulation for use of any one of claims 1-8, wherein the migalastat or salt thereof is administered for at least 12 months.

10. The formulation for use of any one of claims 1-9, wherein the migalastat or salt thereof is administered for at least 24 months.

11. The formulation for use of any one of claims 1-10, wherein the patient is an enzyme replacement therapy (ERT)-naïve patient.

12. The formulation for use of any one of claims 1-11 wherein the administration of migalastat or a salt thereof provides an average increase in MWFS in a group of ERT-naïve patients with impaired MWFS of at least about 1% after 24 months of administration of migalastat or a salt thereof.

13. The formulation for use of any one of claims 1-10, wherein the patient is an ERT-experienced patient.

14. The formulation for use of any one of claims 1-13, wherein the patient has a HEK assay amenable mutation in α-galactosidase A.

15. The formulation for use of any one of claims 2-14, wherein administration of the migalastat or a salt thereof normalizes the patient's MWFS.

## Patentansprüche

1. Formulierung, umfassend eine wirksame Menge Migalastat oder eines Salz davon zur Verwendung bei der Erhöhung der auf die Wandmitte bezogenen linksventrikulären Verkürzungsfraktion (midwall fractional shortening, MWFS) bei einem Patienten mit Morbus Fabry, wobei die wirksame Menge 100 mg bis 150 mg Äquivalent der freien Base (FBE) beträgt und die Formulierung dem Patienten jeden zweiten Tag verabreicht wird.

2. Formulierung zur Verwendung nach Anspruch 1, wobei der Patient vor Beginn der Verabreichung von Migalastat oder einem Salz davon eine beeinträchtigte MWFS aufweist.

3. Formulierung zur Verwendung nach Anspruch 1 oder 2, wobei das Migalastat oder ein Salz davon die α-Galactosidase-A-Aktivität erhöht.

4. Formulierung zur Verwendung nach einem der Ansprüche 1 bis 3, wobei die wirksame Menge 123 mg FBE beträgt.

5. Formulierung zur Verwendung nach einem der Ansprüche 1 bis 4, wobei die wirksame Menge 123 mg Migalastat freie Base beträgt.

6. Formulierung zur Verwendung nach einem der Ansprüche 1 bis 4, wobei die wirksame Menge 150 mg Migalastat-Hydrochlorid beträgt.

7. Formulierung zur Verwendung nach einem der Ansprüche 1 bis 6, wobei die Formulierung eine orale Darreichungsform umfasst.

8. Formulierung zur Verwendung nach Anspruch 7, wobei die orale Darreichungsform eine Tablette, eine Kapsel oder eine Lösung umfasst.

9. Formulierung zur Verwendung nach einem der Ansprüche 1 bis 8, wobei das Migalastat oder ein Salz davon für mindestens 12 Monate verabreicht wird.

10. Formulierung zur Verwendung nach einem der Ansprüche 1 bis 9, wobei das Migalastat oder ein Salz davon für mindestens 24 Monate verabreicht wird.

11. Formulierung zur Verwendung nach einem der Ansprüche 1 bis 10, wobei es sich bei dem Patient um einen Patienten handelt, dessen Erkrankung noch nicht mit einer Enzymersatztherapie (ERT) behandelt wurde.

12. Formulierung zur Verwendung nach einem der Ansprüche 1 bis 11, wobei die Verabreichung von Migalastat oder eines Salzes davon einen durchschnittlichen Anstieg der MWFS in einer Gruppe von ERT-Patienten mit beeinträchtigter MWFS von mindestens etwa 1 % nach 24 Monaten der Verabreichung von Migalastat oder eines Salzes davon ergibt.

13. Formulierung zur Verwendung nach einem der Ansprüche 1 bis 10, wobei es sich bei dem Patient um einen Patienten handelt, dessen Erkrankung bereits mit ERT behandelt wurde.

14. Formulierung zur Verwendung nach einem der Ansprüche 1 bis 13, wobei der Patient eine Mutation in α-Galactosidase A aufweist, die für den HEK-Assay zugänglich ist.

15. Formulierung zur Verwendung nach einem der Ansprüche 2 bis 14, wobei die Verabreichung von Migalastat oder eines Salzes davon die MWFS des Patienten normalisiert.

## Revendications

1. Formulation comprenant une quantité efficace de migalastat ou d'un sel de celui-ci pour une utilisation pour augmenter la fraction de raccourcissement endocardique (FRE) chez un patient ayant la maladie de Fabry, la quantité efficace étant de 100 mg à 150 mg d'équivalent base libre (EBL), la formulation étant administrée au patient une fois par jour.

2. Formulation pour une utilisation selon la revendication 1, le patient présentant une dégradation de la FRE avant le début de l'administration du migalastat ou du sel de celui-ci.

3. Formulation pour une utilisation selon la revendication 1 ou 2, le migalastat ou le sel de celui-ci renforçant l'activité d'a-galactosidase A.

4. Formulation pour une utilisation selon l'une quelconque des revendications 1 à 3, la quantité efficace étant de 123 mg d'EBL.

5. Formulation pour une utilisation selon l'une quelconque des revendications 1 à 4, la quantité efficace étant de 123 mg de migalastat base libre.

6. Formulation pour une utilisation selon l'une quelconque des revendications 1 à 4, la quantité efficace étant de 150 mg de chlorhydrate de migalastat.

7. Formulation pour une utilisation selon l'une quelconque des revendications 1 à 6, la formulation comprenant une forme posologique orale.

8. Formulation pour une utilisation selon la revendication 7, la forme posologique orale comprenant un comprimé, une gélule ou une solution.

9. Formulation pour une utilisation selon l'une quelconque des revendications 1 à 8, le migalastat ou le sel de celui-ci étant administré pendant au moins 12 mois.

10. Formulation pour une utilisation selon l'une quelconque des revendications 1 à 9, le migalastat ou le sel de celui-ci étant administré pendant au moins 24 mois.

11. Formulation pour une utilisation selon l'une quelconque des revendications 1 à 10, le patient étant un patient naïf vis-à-vis d'une enzymothérapie de remplacement (ETR).

12. Formulation pour une utilisation selon l'une quelconque des revendications 1 à 11, l'administration de migalastat ou d'un sel de celui-ci assurant une augmentation moyenne de la FRE dans un groupe de patients ETR-naïfs présentant une dégradation de la FRE, d'au moins environ 1 % après 24 mois d'administration du migalastat ou d'un sel de celui-ci.

13. Formulation pour une utilisation selon l'une quelconque des revendications 1 à 10, le patient étant un patient ayant déjà subi une ETR.

14. Formulation pour une utilisation selon l'une quelconque des revendications 1 à 13, le patient présentant une mutation sensible à un dosage de HEK dans l'α-galactosidase A.

15. Formulation pour une utilisation selon l'une quelconque des revendications 2 à 14, l'administration de migalastat ou d'un sel de celui-ci normalisant la FRE du patient.
